(19) Europäisches Patentamt / European Patent Office / Office européen des brevets

(11) **EP 4 317 439 A1**

(12) **EUROPEAN PATENT APPLICATION**
published in accordance with Art. 153(4) EPC

(43) Date of publication:
**07.02.2024 Bulletin 2024/06**

(21) Application number: **22781256.7**

(22) Date of filing: **31.03.2022**

(51) International Patent Classification (IPC):
*C12N 15/62* (2006.01)    *A61K 35/17* (2015.01)
*A61P 35/00* (2006.01)    *C07K 14/725* (2006.01)
*C07K 16/28* (2006.01)    *C07K 19/00* (2006.01)
*C12N 5/10* (2006.01)    *C12N 15/12* (2006.01)
*C12N 15/13* (2006.01)    *C12N 15/63* (2006.01)

(52) Cooperative Patent Classification (CPC):
**A61K 35/17; A61P 35/00; C07K 14/435;
C07K 14/705; C07K 16/00; C07K 16/28;
C07K 19/00; C12N 5/10; C12N 15/62; C12N 15/63**

(86) International application number:
**PCT/JP2022/016625**

(87) International publication number:
**WO 2022/211046 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021  JP 2021059086**

(71) Applicants:
  • **Shionogi & Co., Ltd**
    **Osaka-shi, Osaka 541-0045 (JP)**
  • **OSAKA UNIVERSITY**
    **Suita-shi**
    **Osaka 565-0871 (JP)**

(72) Inventors:
  • **YOSHIDA, Tetsuya**
    **Osaka-shi, Osaka 541-0045 (JP)**
  • **YOSHIKAWA, Mai**
    **Osaka-shi, Osaka 541-0045 (JP)**
  • **HARUNA, Miya**
    **Osaka-shi, Osaka 541-0045 (JP)**

  • **NAGIRA, Morio**
    **Osaka-shi, Osaka 541-0045 (JP)**
  • **TAKAHASHI, Koji**
    **Osaka-shi, Osaka 541-0045 (JP)**
  • **HAYASHIDA, Marina**
    **Osaka-shi, Osaka 541-0045 (JP)**
  • **MIWA, Hiroto**
    **Osaka-shi, Osaka 541-0045 (JP)**
  • **SONODA, Yudai**
    **Osaka-shi, Osaka 541-0045 (JP)**
  • **OHKURA, Naganari**
    **Suita-shi, Osaka 565-0871 (JP)**
  • **SAKAGUCHI, Shimon**
    **Suita-shi, Osaka 565-0871 (JP)**
  • **WADA, Hisashi**
    **Suita-shi, Osaka 565-0871 (JP)**

(74) Representative: **Mewburn Ellis LLP**
    **Aurora Building**
    **Counterslip**
    **Bristol BS1 6BX (GB)**

Remarks:
    The complete document including Reference
    Table(s) and the Sequence Listing(s) can be
    downloaded from the EPO website

(54) **CHIMERIC ANTIGEN RECEPTOR THAT RECOGNIZES CCR8 AS ANTIGEN**

(57)    The chimeric antigen receptor that recognizes CCR8 as an antigen of the present invention has cytotoxic activity against CCR8-expressing cells by being expressed in effector cells.

**EP 4 317 439 A1**

**Description**

[TECHNICAL FIELD]

The present invention relates to a chimeric antigen receptor that recognizes CCR8 as an antigen, a cell expressing the chimeric antigen receptor, and a pharmaceutical composition containing the cell.

[BACKGROUND ART]

Potent negative regulation mechanisms, including immunosuppression, mediated by regulatory T cells (Treg cells) in the tumor microenvironment are major obstacles to the treatment of tumors (Non-patent Document 1).

For example, CD4-positive Treg cells which infiltrate tumors may be able to strongly inhibit anti-tumor immune response and may become a major obstacle to effective cancer treatment.

Tumor immunosuppression mediated by CD4-positive FoxP3-positive Treg cells has been sufficiently demonstrated in animal tumor models. It has been reported that systemic (including intratumoral) Treg cell removal produces an anti-tumor effect, wherein the removal of approximately 50% tumor-infiltrating Treg cells is not effective (Non-patent Document 2).

It has been reported that the increased ratio of CD4-positive CD25-positive Treg cells (cell population including Treg cells) to the whole CD4-positive T cell population in humans is intratumorally detected in patients with various cancers including lung, breast, and ovary tumors, and the abundance ratio correlates negatively with the survival probabilities of the patients (Non-patent Documents 3 to 8).

CCR8, also previously called CY6, CKR-L1 or TER1, is a G protein-coupled 7-transmembrane CC chemokine receptor protein expressed in the thymus, the spleen, etc. A gene encoding this protein resides on human chromosome 3p21. Human CCR8 consists of 355 amino acids (Non-patent Document 9). CCL1 is known as an endogenous ligand for CCR8 (Non-patent Document 10). Human CCR8 cDNA is constituted by the nucleotide sequence represented by GenBank ACC No. NM_005201.3, and mouse CCR8 cDNA is constituted by the nucleotide sequence represented by GenBank ACC No. NM_007720.2.

CCR8 is also specifically expressed in tumor-infiltrating Treg cells, and it has been demonstrated that, when breast cancer cells were inoculated in CCR8-deficient and wild-type mice, breast cancer proliferation and metastasis were more suppressed in the CCR8-deficient mouse compared to the wild-type mice (Patent Document 1 and Non-patent Document 11). Non-patent Documents 12 to 16 also describe that CCR8 is involved in the pathology of cancer. Furthermore, it has been disclosed that anti-CCR8 antibody administration to a cancer model animal showed an anti-tumor effect (Patent Documents 2 to 12).

A chimeric antigen receptor (CAR) comprising an extracellular region that binds to a tumor antigen, a transmembrane region, and an intracellular region, wherein the intracellular region comprises an intracellular signal region of a signaling molecule such as a T cell receptor, expressed in a lymphocyte that functions as an effector cell such as a T cell or an NK cell, is known to be useful for cancer treatment because the chimeric antigen receptor exhibits cytotoxic activity against a tumor expressing the antigen when expressed in T cells (Non-patent Documents 17 and 18) or NK cells (Non-patent Documents 19 and 20).

However, it has not been shown so far that a chimeric antigen receptor comprising an extracellular region that binds to an antigen expressed in a tumor-infiltrating Treg cell, such as CCR8, a transmembrane region, and an intracellular region, wherein the intracellular region comprises an intracellular signal region of a signaling molecule expressed in a lymphocyte that functions as an effector cell such as a T cell or an NK cell, is useful for cancer treatment.

[PRIOR ART REFERENCES]

[Patent Documents]

[Patent Document 1] US 10087259
[Patent Document 2] WO 2018/181425
[Patent Document 3] WO 2018/112032
[Patent Document 4] WO 2020/138489
[Patent Document 5] WO 2021/142002
[Patent Document 6] WO 2021/152186
[Patent Document 7] WO 2021/163064
[Patent Document 8] WO 2021/178749

[Patent Document 9] WO 2021/194942
[Patent Document 10] WO 2021/260208
[Patent Document 11] WO 2021/260209
[Patent Document 12] WO 2021/260210

[Non-patent Documents]

[Non-patent Document 1] Nat. Rev. Immunol., 2006, Vol. 6, No. 4, p.295-307
[Non-patent Document 2] Eur. J. Immunol., 2010, Vol. 40, p.3325-3335
[Non-patent Document 3] J. Clin. Oncol., 2006, Vol. 24, p.5373-5380
[Non-patent Document 4] Nat. Med., 2004, Vol. 10, p.942-949
[Non-patent Document 5] J. Clin. Oncol., 2007, Vol. 25, p.2586-2593
[Non-patent Document 6] Cancer, 2006, Vol. 107, p.2866-2872
[Non-patent Document 7] Eur. J. Cancer, 2008, Vol. 44, p.1875-1882
[Non-patent Document 8] Cell. Mol. Immunol. 2011, Vol. 8, p.59-66
[Non-patent Document 9] J. Immunol., 1996, Vol. 157, No. 7, p. 2759-63
[Non-patent Document 10] J. Biol. Chem., 1997, Vol. 272, No. 28, p. 17251-4
[Non-patent Document 11] Cancer Res., 2016, Vol. 76, No. 4, Supp.1, P4-04-11
[Non-patent Document 12] Immunity, 2016, Vol. 45, p1122-1134
[Non-patent Document 13] Immunity, 2016, Vol. 45, p1135-1147
[Non-patent Document 14] Proc. Natl. Acad. Sci. USA, 2018, Vol. 115, No. 45, p10672-10681
[Non-patent Document 15] Targeting of CCR8 induces antitumor activity as a monotherapy that is further enhanced in combination with a Listeria-based immunotherapy, ASCO 2018, Daniel O Villarreal, et al. https://www.advaxis.com/wp-content/uploads/2018/04/KeystonePoster CCR8-combo-posterFINAL.pdf
[Non-patent Document 16] Cancer Res., 2018, Vol. 78, No. 18, p5340-5348
[Non-patent Document 17] Proc. Natl. Acad. Sci. USA, 1993, Vol. 90, No. 2, p720-724
[Non-patent Document 18] Cell Res., 2017, Vol. 27, p38-58
[Non-patent Document 19] Cell Stem Cell, 2018, Vol. 23, No. 2, p181-192
[Non-patent Document 20] J. Hematol. Oncol., 2019, Vol. 12, No. 1, 49

[SUMMARY OF THE INVENTION]

[PROBLEMS TO BE SOLVED BY THE INVENTION]

An object of the present invention is to provide a chimeric antigen receptor that recognizes CCR8 as an antigen. A further object of the present invention is to provide a cell expressing a chimeric antigen receptor that recognizes CCR8 as an antigen, which is useful for cancer treatment.

[MEANS FOR SOLVING THE PROBLEMS]

The present inventors have found, as a result of intensive studies, a chimeric antigen receptor that recognizes CCR8 as an antigen. The present inventors have further found that a cell expressing the chimeric antigen receptor that recognizes CCR8 as an antigen of the present invention has cytotoxic activity against CCR8-expressing cells. In other words, the cell expressing the chimeric antigen receptor that recognizes CCR8 as an antigen of the present invention is useful for cancer treatment.
Specifically, the present invention relates to the following.

(1) A chimeric antigen receptor comprising an extracellular region that binds to CCR8, a transmembrane region, and an intracellular region, wherein the intracellular region comprises an intracellular signal region of a signaling molecule expressed in a lymphocyte that functions as an effector cell.
(2) The chimeric antigen receptor according to (1), wherein the extracellular region that binds to CCR8 comprises an anti-CCR8-scFv region.
(3) The chimeric antigen receptor according to (2), wherein the anti-CCR8-scFv region comprises:

1) a light chain variable region including

CDR1 having the amino acid sequence of SEQ ID NO: 24,
CDR2 having the amino acid sequence of SEQ ID NO: 25, and
CDR3 having the amino acid sequence of SEQ ID NO: 26, and
a heavy chain variable region including
CDR1 having the amino acid sequence of SEQ ID NO: 27,
CDR2 having the amino acid sequence of SEQ ID NO: 28, and
CDR3 having the amino acid sequence of SEQ ID NO: 29;

2) a light chain variable region including

CDR1 having the amino acid sequence of SEQ ID NO: 30,
CDR2 having the amino acid sequence of SEQ ID NO: 31, and
CDR3 having the amino acid sequence of SEQ ID NO: 32, and a heavy chain variable region including
CDR1 having the amino acid sequence of SEQ ID NO: 33, CDR2 having the amino acid sequence of SEQ ID NO: 28, and CDR3 having the amino acid sequence of SEQ ID NO: 34;

3) a light chain variable region including

CDR1 having the amino acid sequence of SEQ ID NO: 35,
CDR2 having the amino acid sequence of SEQ ID NO: 36, and
CDR3 having the amino acid sequence of SEQ ID NO: 37, and
a heavy chain variable region including
CDR1 having the amino acid sequence of SEQ ID NO: 38,
CDR2 having the amino acid sequence of SEQ ID NO: 39, and
CDR3 having the amino acid sequence of SEQ ID NO: 40;

4) a light chain variable region including

CDR1 having the amino acid sequence of SEQ ID NO: 30,
CDR2 having the amino acid sequence of SEQ ID NO: 41, and
CDR3 having the amino acid sequence of SEQ ID NO: 32, and
a heavy chain variable region including
CDR1 having the amino acid sequence of SEQ ID NO: 42,
CDR2 having the amino acid sequence of SEQ ID NO: 28, and
CDR3 having the amino acid sequence of SEQ ID NO: 43;

5) a light chain variable region including

CDR1 having the amino acid sequence of SEQ ID NO: 44,
CDR2 having the amino acid sequence of SEQ ID NO: 45, and
CDR3 having the amino acid sequence of SEQ ID NO: 46, and
a heavy chain variable region including
CDR1 having the amino acid sequence of SEQ ID NO: 47,
CDR2 having the amino acid sequence of SEQ ID NO: 28, and
CDR3 having the amino acid sequence of SEQ ID NO: 48; or

6) a light chain variable region including

CDR1 having the amino acid sequence of SEQ ID NO: 56,
CDR2 having the amino acid sequence of SEQ ID NO: 57, and
CDR3 having the amino acid sequence of SEQ ID NO: 58, and
a heavy chain variable region including
CDR1 having the amino acid sequence of SEQ ID NO: 59,
CDR2 having the amino acid sequence of SEQ ID NO: 60, and
CDR3 having the amino acid sequence of SEQ ID NO: 61.

(4) The chimeric antigen receptor according to (3), wherein the anti-CCR8-scFv region comprises:

1) a light chain variable region including

CDR1 having the amino acid sequence of SEQ ID NO: 24,
CDR2 having the amino acid sequence of SEQ ID NO: 25, and
CDR3 having the amino acid sequence of SEQ ID NO: 26, and
a heavy chain variable region including
CDR1 having the amino acid sequence of SEQ ID NO: 27,
CDR2 having the amino acid sequence of SEQ ID NO: 28, and
CDR3 having the amino acid sequence of SEQ ID NO: 29;

2) a light chain variable region including

CDR1 having the amino acid sequence of SEQ ID NO: 30,
CDR2 having the amino acid sequence of SEQ ID NO: 31, and
CDR3 having the amino acid sequence of SEQ ID NO: 32, and
a heavy chain variable region including
CDR1 having the amino acid sequence of SEQ ID NO: 33,
CDR2 having the amino acid sequence of SEQ ID NO: 28, and
CDR3 having the amino acid sequence of SEQ ID NO: 34; or

4) a light chain variable region including

CDR1 having the amino acid sequence of SEQ ID NO: 30,
CDR2 having the amino acid sequence of SEQ ID NO: 41, and
CDR3 having the amino acid sequence of SEQ ID NO: 32, and
a heavy chain variable region including
CDR1 having the amino acid sequence of SEQ ID NO: 42,
CDR2 having the amino acid sequence of SEQ ID NO: 28, and
CDR3 having the amino acid sequence of SEQ ID NO: 43.

(5) The chimeric antigen receptor according to (4), wherein the anti-CCR8-scFv region comprises:

a light chain variable region including
CDR1 having the amino acid sequence of SEQ ID NO: 24,
CDR2 having the amino acid sequence of SEQ ID NO: 25, and
CDR3 having the amino acid sequence of SEQ ID NO: 26, and
a heavy chain variable region including
CDR1 having the amino acid sequence of SEQ ID NO: 27,
CDR2 having the amino acid sequence of SEQ ID NO: 28, and
CDR3 having the amino acid sequence of SEQ ID NO: 29.

(6) The chimeric antigen receptor according to (4), wherein the anti-CCR8-scFv region comprises:

a light chain variable region including
CDR1 having the amino acid sequence of SEQ ID NO: 30,
CDR2 having the amino acid sequence of SEQ ID NO: 31, and
CDR3 having the amino acid sequence of SEQ ID NO: 32, and
a heavy chain variable region including
CDR1 having the amino acid sequence of SEQ ID NO: 33,
CDR2 having the amino acid sequence of SEQ ID NO: 28, and
CDR3 having the amino acid sequence of SEQ ID NO: 34.

(7) The chimeric antigen receptor according to (2) or (3), wherein the anti-CCR8-scFv region comprises:

1) a light chain variable region having the amino acid sequence of SEQ ID NO: 12, and
a heavy chain variable region having the amino acid sequence of SEQ ID NO: 13;
2) a light chain variable region having the amino acid sequence of SEQ ID NO: 14, and
a heavy chain variable region having the amino acid sequence of SEQ ID NO: 15; or

3) a light chain variable region having the amino acid sequence of SEQ ID NO: 16, and
a heavy chain variable region having the amino acid sequence of SEQ ID NO: 17.

(8) The chimeric antigen receptor according to (7), wherein the anti-CCR8-scFv region comprises:

1) a light chain variable region having the amino acid sequence of SEQ ID NO: 12, and
a heavy chain variable region having the amino acid sequence of SEQ ID NO: 13; or
2) a light chain variable region having the amino acid sequence of SEQ ID NO: 14, and
a heavy chain variable region having the amino acid sequence of SEQ ID NO: 15.

(9) The chimeric antigen receptor according to (8), wherein the anti-CCR8-scFv region comprises:

a light chain variable region having the amino acid sequence of SEQ ID NO: 12, and
a heavy chain variable region having the amino acid sequence of SEQ ID NO: 13.

(10) The chimeric antigen receptor according to (2), wherein the anti-CCR8-scFv region comprises:

a light chain variable region having the amino acid sequence of SEQ ID NO: 14, and
a heavy chain variable region having the amino acid sequence of SEQ ID NO: 15.

(11) The chimeric antigen receptor according to any one of (2) to (10), wherein the light chain variable region and the heavy chain variable region are linked via the linker sequence of SEQ ID NO: 2 in the anti-CCR8-scFv region.
(12) The chimeric antigen receptor according to any one of (2) to (11), wherein the extracellular region that binds to CCR8 comprises a CD8A signal sequence region.
(13) The chimeric antigen receptor according to (12), wherein the CD8A signal sequence region is present on an N-terminal side of the anti-CCR8-scFv region.
(14) The chimeric antigen receptor according to (12) or (13), wherein the CD8A signal sequence region has the amino acid sequence of SEQ ID NO: 1.
(15) The chimeric antigen receptor according to any one of (2) to (14), wherein the anti-CCR8-scFv region and the transmembrane region are linked via a spacer sequence.
(16) The chimeric antigen receptor according to (15), wherein the spacer sequence comprises a Flag epitope-containing sequence region.
(17) The chimeric antigen receptor according to (16), wherein the Flag epitope-containing sequence region has the amino acid sequence of SEQ ID NO: 3.
(18) The chimeric antigen receptor according to any one of (15) to (17), wherein the spacer sequence comprises a CD8A hinge region.
(19) The chimeric antigen receptor according to (18), wherein the CD8A hinge region has the amino acid sequence of SEQ ID NO: 4.
(20) The chimeric antigen receptor according to any one of (1) to (19), wherein the transmembrane region comprises a CD8A transmembrane region.
(21) The chimeric antigen receptor according to (20), wherein the CD8A transmembrane region has the amino acid sequence of SEQ ID NO: 5.
(22) The chimeric antigen receptor according to any one of (1) to (19), wherein the transmembrane region comprises a CD28 transmembrane region and the intracellular region comprises a CD28 intracellular signal region.
(23) The chimeric antigen receptor according to (22), wherein the CD28 transmembrane region has the amino acid sequence of SEQ ID NO: 6, and the CD28 intracellular signal region has the amino acid sequence of SEQ ID NO: 7.
(24) The chimeric antigen receptor according to any one of (1) to (23), wherein the intracellular region comprises at least one of intracellular signal regions of 4-1BB, 2B4, CD3£, and DAP10.
(25) The chimeric antigen receptor according to (24), wherein the intracellular region comprises at least two of intracellular signal regions of 4-1BB, 2B4, CD3£, and DAP 10.
(26) The chimeric antigen receptor according to (25), wherein the intracellular region comprises intracellular signal regions of 4-1BB and CD3£.
(27) The chimeric antigen receptor according to (25), wherein the intracellular region comprises intracellular signal regions of 2B4 and CD3£.
(28) The chimeric antigen receptor according to any one of (24) to (27), wherein the intracellular region comprises a 4-1BB intracellular signal region, and the 4-1BB intracellular signal region has the amino acid sequence of SEQ ID NO: 8.
(29) The chimeric antigen receptor according to any one of (24) to (27), wherein the intracellular region comprises a 2B4 intracellular signal region, and the 2B4 intracellular signal region has the amino acid sequence of SEQ ID NO: 9.

(30) The chimeric antigen receptor according to any one of (24) to (27), wherein the intracellular region comprises a CD3£ intracellular signal region, and the CD3£ intracellular signal region has the amino acid sequence of SEQ ID NO: 10.

(31) The chimeric antigen receptor according to (24) to (27), wherein the intracellular region comprises a DAP10 intracellular signal region, and the DAP10 intracellular signal region has the amino acid sequence of SEQ ID NO: 11.

(32) An effector cell expressing the chimeric antigen receptor according to any one of (1) to (31).

(33) The cell according to (32), wherein the effector cell is an NK cell.

(34) The cell according to (32), wherein the effector cell is a T cell.

(35) The cell according to (32), wherein the effector cell is a macrophage.

(36) The cell according to any one of (32) to (35), wherein the effector cell is derived from peripheral blood.

(37) The cell according to any one of (32) to (35), wherein the effector cell is differentiated from a stem cell.

(38) The cell according to (36), wherein the stem cell is an iPS cell.

(39) A pharmaceutical composition comprising the cell according to any one of (32) to (38).

(40) The pharmaceutical composition according to (39), for use in treating cancer.

(41) The pharmaceutical composition according to (40), having an effect of depleting tumor-infiltrating Treg cells expressing CCR8.

(42) The pharmaceutical composition according to (40), having an effect of depleting tumor cells expressing CCR8.

(43) A polynucleotide encoding an amino acid sequence of the chimeric antigen receptor according to any one of (1) to (31).

(44) An expression vector comprising the polynucleotide according to (43).

[EFFECTS OF THE INVENTION]

Since the cell expressing the chimeric antigen receptor that recognizes CCR8 as an antigen of the present invention has cytotoxic activity against CCR8-expressing cells, the cell is very useful as a medicament, particularly as a medicament for the treatment or prevention of CCR8-related diseases.

[BRIEF DESCRIPTION OF DRAWINGS]

Figure 1 shows amino acid sequences in a light chain variable region (VL) and a heavy chain variable region (VH) of an anti-human CCR8 antibody (αCCR8-1 to 7). In the amino acid sequences, bold letters mean CDR1, CDR2, and CDR3 in order.

Figure 2 shows a structure of a chimeric antigen receptor that recognizes CCR8 as an antigen (anti-CCR8-CAR).

Figure 3 shows evaluation of cytotoxic activity of KHYG-1 cells into which mRNAs of anti-CCR8-CARs (αCCR8-1-CAR and αCCR8-2-CAR) were introduced using human CCR8-expressing Rat-1 cells as target cells.

Figure 4 shows evaluation of cytotoxic activity of KHYG-1 cells into which mRNA of each anti-CCR8-CAR having the same anti-CCR8-scFv region but a different transmembrane region and a different intracellular region (αCCR8-2-CAR-1 to 6) was introduced using human CCR8-expressing Rat-1 cells as target cells.

Figure 5 shows evaluation of cytotoxic activity of KHYG-1 cells into which mRNA of each anti-CCR8-CAR having the same anti-CCR8-scFv region but a different transmembrane region and a different intracellular region (αCCR8-2-CAR-2 and αCCR8-2-CAR-12) was introduced using human CCR8-expressing Rat-1 cells as target cells.

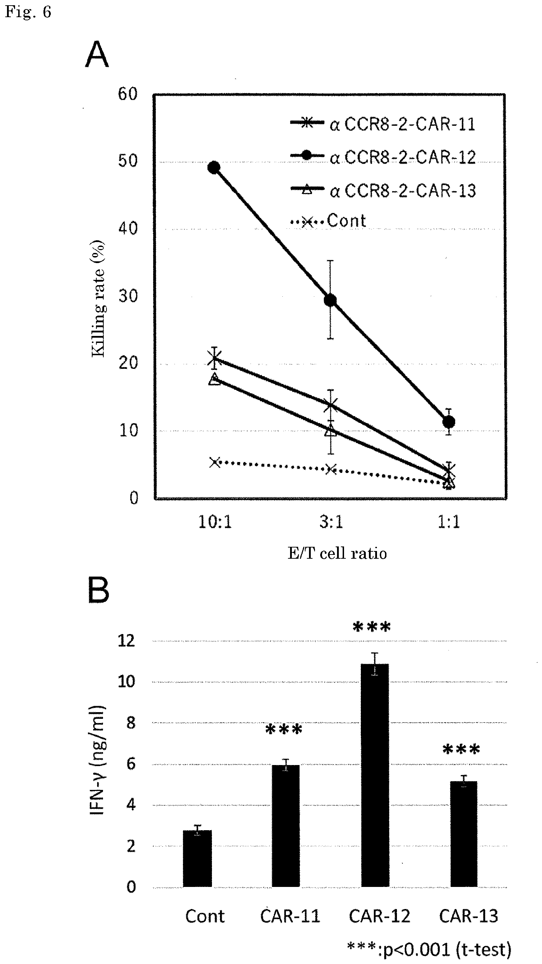

Figure 6(A) shows evaluation of cytotoxic activity of KHYG-1 cells into which mRNA of each anti-CCR8-CAR having the same anti-CCR8-scFv region but a different transmembrane region and a different intracellular region (αCCR8-2-CAR-11 to 13) was introduced using human CCR8-expressing Rat-1 cells as target cells. Figure 6(B) shows evaluation of an amount of interferon-γ secreted into a culture supernatant by KHYG-1 cells into which mRNA of each anti-CCR8-CAR having the same anti-CCR8-scFv region but a different transmembrane region and a different intracellular region (αCCR8-2-CAR-11 to 13) was introduced.

Figure 7 shows evaluation of cytotoxic activity of KHYG-1 cells into which mRNA of each anti-CCR8-CAR having a different anti-CCR8-scFv region and the same transmembrane region and the same intracellular region (αCCR8-1-CAR to αCCR8-6-CAR) was introduced using human CCR8-expressing Rat-1 cells as target cells.

Figure 8 shows evaluation of cytotoxic activity of KHYG-1 cells into which mRNAs of anti-CCR8-CARs (αCCR8-1-CAR and αCCR8-2-CAR) were introduced using human CCR8-expressing RAMOS cells (C5 cells and C6 cells) as target cells.

Figure 9(A) shows examples of analysis of cytotoxic activity of human CCR8-expressing Treg cells by a flow cytometer. Figure 9(B) shows evaluation of cytotoxic activity of KHYG-1 cells into which mRNAs of a negative control and αCCR8-2-CAR were introduced using human CCR8-expressing Treg cells as target cells.

Figure 10 shows evaluation of cytotoxic activity of KHYG-1 cells into which mRNA of an anti-CCR8-CAR (αCCR8-7-CAR) was introduced using human CCR8-expressing Rat-1 cells as target cells.

Figure 11 shows evaluation of cytotoxic activity of human peripheral blood-derived NK cells into which mRNA of an anti-CCR8-CAR (αCCR8-2-CAR-2) was introduced using human CCR8-expressing Rat-1 cells as target cells.

Figure 12 shows evaluation of cytotoxic activity of human peripheral blood-derived T cells into which mRNAs of anti-CCR8-CARs (αCCR8-2-CAR-2 and αCCR8-2-CAR-12) were introduced using ATL cell line-derived ATN-1 cells as target cells.

Figure 13 shows evaluation of cytotoxic activity of human peripheral blood-derived T cells into which mRNAs of anti-CCR8-CARs (αCCR8-2-CAR-2 and αCCR8-2-CAR-12) were introduced using ALL cell line-derived TALL-1 cells as target cells.

Figure 14 shows evaluation of cytotoxic activity of KHYG-1 cells into which mRNA of an anti-CCR8-CAR (αCCR8-2-CAR-12) was introduced using ATL cell line-derived ATN-1 cells as target cells.

Figure 15 shows evaluation of inhibitory activity of an anti-CCR8 antibody against cytotoxic activity of KHYG-1 cells into which mRNA of an anti-CCR8-CAR (αCCR8-2-CAR-12) was introduced using ATL cell line-derived ATN-1 cells as target cells.

Figure 16 shows evaluation of an anti-tumor effect of KHYG-1 cells into which mRNA of an anti-CCR8-CAR (αCCR8-2-CAR-12) was introduced in mice inoculated with human CCR8-expressing Rat-1 cells.

Figure 17(A) shows evaluation of cytotoxic activity of human iPS cell-derived NK cells into which mRNA of an anti-CCR8-CAR (αCCR8-2-CAR-2) was introduced using human CCR8-expressing Rat-1 cells as target cells. Figure 17(B) shows evaluation of cytotoxic activity of human iPS cell-derived macrophages into which mRNA of an anti-CCR8-CAR (αCCR8-2-CAR-2) was introduced using human CCR8-expressing Rat-1 cells as target cells.

Figure 18 shows evaluation of cytotoxic activity of KHYG-1 cells into which mRNA of an anti-CCR8-CAR (αCCR8-2-CAR-2) was introduced using human ovarian cancer-derived tumor-infiltrating Treg cells as target cells.

Figure 19 shows evaluation of cytotoxic activity of human peripheral blood-derived NK cells into which mRNA of an anti-CCR8-CAR (αCCR8-2-CAR-2) was introduced using human ovarian cancer-derived tumor-infiltrating Treg cells as target cells.

Figure 20 shows evaluation of cytotoxic activity of human peripheral blood-derived NK cells into which mRNA of an anti-CCR8-CAR (αCCR8-2-CAR-2) was introduced using human colorectal cancer-derived tumor-infiltrating Treg cells as target cells.

Figure 21 shows evaluation of an anti-tumor effect of KHYG-1 cells into which mRNA of an anti-CCR8-CAR (αCCR8-2-CAR-12) was introduced in mice inoculated with ATL cell line-derived ATN-1 cells.

Figure 22 shows an anti-tumor effect of KHYG-1 cells into which mRNA of an anti-CCR8-CAR (αCCR8-2-CAR-12) was introduced in mice inoculated with ATL cell line-derived ATN-1 cells by luminescence imaging data.

Figure 23 shows evaluation of an anti-tumor effect of KHYG-1 cells into which mRNA of an anti-CCR8-CAR (αCCR8-2-CAR-12) was introduced in mice inoculated with colorectal cancer-derived CT26 cells.

Figure 24 shows evaluation of cytotoxic activity of KHYG-1 cells into which mRNA of an anti-CCR8-CAR (αCCR8-2-CAR-12) was introduced against tumor-infiltrating Treg cells in mice inoculated with colorectal cancer-derived CT26 cells.

[MODE FOR CARRYING OUT THE INVENTION]

The term used in the present description is used in the meaning usually used in the art, unless specifically mentioned.

The chimeric antigen receptor of the present invention comprises an extracellular region that binds to CCR8, a transmembrane region, and an intracellular region, wherein the intracellular region comprises an intracellular signal region of a signaling molecule expressed in a lymphocyte that functions as an effector cell.

The chimeric antigen receptor of the present invention can be expressed by determining an entire amino acid sequence on the basis of amino acid sequence information on the "extracellular region that binds to CCR8", the "transmembrane region", and the "intracellular region" to construct an expression vector, and transforming an effector cell using the expression vector.

The "extracellular region that binds to CCR8" means an extracellular region comprising a fragment of an anti-CCR8 antibody that is part of the anti-CCR8 antibody and that specifically binds to CCR8 similarly to the anti-CCR8 antibody.

The "extracellular region that binds to CCR8" may further comprise a signal sequence region at an N-terminus. Examples of the signal sequence region include a CD8A signal sequence region, and a CD28 signal sequence region, and a CD8A signal sequence is preferred. The CD8A signal sequence region preferably has the amino acid sequence of SEQ ID NO: 1.

Antibody-producing techniques known in the art can be used for the anti-CCR8 antibody. Examples of the techniques include a method described in Immunochemistry in Practice (Blackwell Scientific Publiations).

Genetically engineered techniques known in the art can also be used. Examples of the techniques include methods described in Molecular Cloning, A Laboratory Manual, Forth Edition, Cold Spring Harbor Laboratory Press (2012), and Current Protocols Essential Laboratory Techniques, Current Protocols (2012).

The amino acid sequence of human CCR8 is shown in UniProtKB/Swiss-Prot: P51685 (SEQ ID NO: 49). The extramembrane domains of human CCR8 correspond to the N-terminal region consisting of amino acids positions 1-35, the loop1 region consisting of amino acids positions 94-107, the loop2 region consisting of amino acids positions 172-202, and the loop3 region consisting of amino acids positions 264-280.

A hybridoma producing the anti-CCR8 antibody can be produced by using, as an immunogen, a human CCR8 protein, a gene encoding the full length of human CCR8, a human CCR8-expressing cell, or the like. When a gene encoding the full length of human CCR8 is used an immunogen, a hybridoma producing the anti-CCR8 antibody can be prepared, for example, by fusing a spleen cell of a mouse which has been DNA-immunized with the gene as an antigen into a mouse myeloma cell.

One aspect of the anti-CCR8 antibody includes a monoclonal antibody having a CDR or a heavy/light chain variable region described in the present description. The antibody or an antibody fragment may be from any class or subclass of immunoglobulin molecule (e.g., IgG, IgE, IgM, IgD, or IgA, preferably, IgG). The antibody or antibody fragment may also be obtained from any species, such as mouse, rat, shark, rabbit, pig, hamster, camel, llama, goat, or human. The antibody or antibody fragment thereof is preferably a mouse-derived monoclonal antibody or a humanized monoclonal antibody.

The anti-CCR8 antibody is characterized in that it inhibits a binding of CCR8 to a CCR8 ligand. The "CCR8 ligand" is not particularly limited as long as it is a substance that binds to CCR8, such as CCL1, CCL8, or CCL18, but is preferably CCL1, CCL18, and particularly preferably CCL1.

The inhibitory ability of binding of CCR8 to a CCR8 ligand can be determined, when the CCR8 ligand is human CCL1, for example, by using human CCR8-expressing 293 cells, determining $Ca^{2+}$ influx by human CCL1 addition, and calculating an IC50 value with setting that the signal when human CCL1 is not added as the inhibition rate of 100% and the signal when human CCL1 is added and the antibody is not added as the inhibition rate of 0%. The inhibitory ability of binding to other CCR8 ligands can also be determined in a similar manner to human CCL1 as described above.

Human CCL1 has an amino acid sequence shown by UniProtKB/Swiss-Prot No. P22362 or the like. Human CCL8 has the amino acid sequence shown by GenBank No. AAI 26243.1 or the like. Human CCL18 has the amino acid sequence shown by GenBank No. EAW80102.1 or the like.

The anti-CCR8 antibody also include a chimeric antibody, a humanized antibody, and a fully human antibody. The humanized monoclonal antibody is useful when administered to humans for therapeutic purposes or the like, because it is less antigenic in humans. A humanized monoclonal antibody is an antibody in which a complementarity determining region (CDR) of a non-human mammal antibody, such as a mouse antibody, is implanted into a framework region (FR) of a human antibody. The FR of a humanized monoclonal antibody is thus derived from a human. Suitable FR can be selected by referring to the documents of Kabat E.A. et al. As the FR in this case, one with which the CDR can form an appropriate antigen binding site is selected. As necessary, amino acids of the FR of a variable region of the antibody may be substituted so that the CDR of the reconstituted humanized monoclonal antibody form an appropriate antigen binding site (Sato, K. et al., Cancer Res. 1993, vol. 53, p. 851). The percentage of amino acids of the FR to be substituted is from 0 to 15%, preferably from 0 to 5%, of the total FR region.

The anti-CCR8 antibody is preferably a CCR8-neutralizing antibody. The CCR8-neutralizing antibody means an antibody having neutralizing activity against CCR8. Whether or not an antibody has neutralizing activity against CCR8 can be determined, for example, by measuring whether or not the antibody inhibits the physiological action of a CCR8 ligand (e.g., CCL1) against CCR8.

The anti-CCR8 antibody is preferably an antibody that strongly recognizes human CCR8. When selecting the antibody that strongly recognizes human CCR8, an antibody or an antibody fragment thereof that more strongly recognizes human CCR8 can be selected by selecting the antibody or an antibody fragment thereof using the strength of neutralizing activity as an index.

As the fragment of the anti-CCR8 antibody comprised in the "extracellular region that binds to CCR8", a single-chain antibody that specifically binds to CCR8 (hereinafter referred to as anti-CCR8-scFv) is preferred.

The anti-CCR8-scFv is a VH-P-VL or VL-P-VH polypeptide in which one VH and one VL are linked with an appropriate linker sequence (hereinafter referred to as P), and is an antibody fragment having CCR8-binding activity. Preferably, the anti-CCR8-scFv is a VH-P-VL polypeptide. The VH and VL contained in scFv used in the present invention may be those of the monoclonal antibody of the present invention. scFv used in the present invention can also be produced by constructing a scFv expression vector using cDNA encoding the VH and VL of the anti-CCR8 antibody according to the present invention and introducing the obtained vector into E. coli, yeast, or an animal cell to express scFv. The linker sequence is preferably the linker sequence of SEQ ID NO: 2.

When the "extracellular region that binds to CCR8" comprises an anti-CCR8-scFv region and a signal sequence region, the signal sequence region is preferably present on an N-terminal side of the anti-CCR8-scFv region.

A peptide comprising a CDR, such as the anti-CCR8-scFv, is composed of at least one or more regions of a CDR of VH or VL. The plurality of CDRs can be bound directly or via an appropriate linker sequence. The peptide comprising a CDR used in the present invention can be produced by constructing a CDR-encoding DNA using cDNA encoding the VH and VL of the monoclonal antibody of the present invention, inserting the DNA into an animal cell expression vector, and introducing the obtained vector into E. coli, yeast, or an animal cell to express the peptide. The peptide comprising a CDR can also be produced by chemical synthesis methods such as the Fmoc method (fluorenylmethyloxycarbonyl method), the tBoc method (t-butyloxycarbonyl method), and the like.

The "extracellular region that binds to CCR8" is characterized in that it binds to human CCR8. In particular, those that specifically bind to human CCR8 are preferred.

The specific binding can be characterized by an equilibrium dissociation constant of at least about $1 \times 10^{-6}$ M or less (for example, the smaller Kd represents the closer binding). The Kd value is preferably $1 \times 10^{-7}$ M or less, more preferably $1 \times 10^{-8}$ M or less, still more preferably $1 \times 10^{-9}$ M or less. Methods for determining whether two molecules specifically bind are well known in the art, and examples thereof include competitive ELISA methods, surface plasmon resonance, and the like other method.

The amino acid sequence of the "extracellular region that binds to CCR8" can be determined using amino acid sequence information on a CDR or a heavy/light chain variable region of the anti-CCR8 antibody described in the present description.

The anti-CCR8-scFv region comprised in the "extracellular region that binds to CCR8" preferably comprises any of the following sequences:

    1) a light chain variable region including

        CDR1 having the amino acid sequence of SEQ ID NO: 24,
        CDR2 having the amino acid sequence of SEQ ID NO: 25, and
        CDR3 having the amino acid sequence of SEQ ID NO: 26, and
        a heavy chain variable region including
        CDR1 having the amino acid sequence of SEQ ID NO: 27,
        CDR2 having the amino acid sequence of SEQ ID NO: 28, and
        CDR3 having the amino acid sequence of SEQ ID NO: 29;

    2) a light chain variable region including

        CDR1 having the amino acid sequence of SEQ ID NO: 30,
        CDR2 having the amino acid sequence of SEQ ID NO: 31, and
        CDR3 having the amino acid sequence of SEQ ID NO: 32, and
        a heavy chain variable region including
        CDR1 having the amino acid sequence of SEQ ID NO: 33,
        CDR2 having the amino acid sequence of SEQ ID NO: 28, and
        CDR3 having the amino acid sequence of SEQ ID NO: 34;

    3) a light chain variable region including

        CDR1 having the amino acid sequence of SEQ ID NO: 35,
        CDR2 having the amino acid sequence of SEQ ID NO: 36, and
        CDR3 having the amino acid sequence of SEQ ID NO: 37, and
        a heavy chain variable region including
        CDR1 having the amino acid sequence of SEQ ID NO: 38,
        CDR2 having the amino acid sequence of SEQ ID NO: 39, and
        CDR3 having the amino acid sequence of SEQ ID NO: 40;

    4) a light chain variable region including

        CDR1 having the amino acid sequence of SEQ ID NO: 30,
        CDR2 having the amino acid sequence of SEQ ID NO: 41, and
        CDR3 having the amino acid sequence of SEQ ID NO: 32, and
        a heavy chain variable region including

CDR1 having the amino acid sequence of SEQ ID NO: 42,
CDR2 having the amino acid sequence of SEQ ID NO: 28, and
CDR3 having the amino acid sequence of SEQ ID NO: 43; or

5) a light chain variable region including

CDR1 having the amino acid sequence of SEQ ID NO: 44,
CDR2 having the amino acid sequence of SEQ ID NO: 45, and
CDR3 having the amino acid sequence of SEQ ID NO: 46, and
a heavy chain variable region including
CDR1 having the amino acid sequence of SEQ ID NO: 47,
CDR2 having the amino acid sequence of SEQ ID NO: 28, and
CDR3 having the amino acid sequence of SEQ ID NO: 48.

6) a light chain variable region including

CDR1 having the amino acid sequence of SEQ ID NO: 56,
CDR2 having the amino acid sequence of SEQ ID NO: 57, and
CDR3 having the amino acid sequence of SEQ ID NO: 58, and
a heavy chain variable region including
CDR1 having the amino acid sequence of SEQ ID NO: 59,
CDR2 having the amino acid sequence of SEQ ID NO: 60, and
CDR3 having the amino acid sequence of SEQ ID NO: 61.

More preferably, the anti-CCR8-scFv region comprises any of the following sequences:

1) a light chain variable region including

CDR1 having the amino acid sequence of SEQ ID NO: 24,
CDR2 having the amino acid sequence of SEQ ID NO: 25, and
CDR3 having the amino acid sequence of SEQ ID NO: 26, and
a heavy chain variable region including
CDR1 having the amino acid sequence of SEQ ID NO: 27,
CDR2 having the amino acid sequence of SEQ ID NO: 28, and
CDR3 having the amino acid sequence of SEQ ID NO: 29;

2) a light chain variable region including

CDR1 having the amino acid sequence of SEQ ID NO: 30,
CDR2 having the amino acid sequence of SEQ ID NO: 31, and
CDR3 having the amino acid sequence of SEQ ID NO: 32, and
a heavy chain variable region including
CDR1 having the amino acid sequence of SEQ ID NO: 33,
CDR2 having the amino acid sequence of SEQ ID NO: 28, and
CDR3 having the amino acid sequence of SEQ ID NO: 34; or

4) a light chain variable region including

CDR1 having the amino acid sequence of SEQ ID NO: 30,
CDR2 having the amino acid sequence of SEQ ID NO: 41, and
CDR3 having the amino acid sequence of SEQ ID NO: 32, and
a heavy chain variable region including
CDR1 having the amino acid sequence of SEQ ID NO: 42,
CDR2 having the amino acid sequence of SEQ ID NO: 28, and
CDR3 having the amino acid sequence of SEQ ID NO: 43.

In addition, the anti-CCR8-scFv region comprised in the "extracellular region that binds to CCR8" preferably comprises any of the following sequences:

1) a light chain variable region having the amino acid sequence of SEQ ID NO: 12, and a heavy chain variable region having the amino acid sequence of SEQ ID NO: 13;
2) a light chain variable region having the amino acid sequence of SEQ ID NO: 14, and a heavy chain variable region having the amino acid sequence of SEQ ID NO: 15; or
3) a light chain variable region having the amino acid sequence of SEQ ID NO: 16, and a heavy chain variable region having the amino acid sequence of SEQ ID NO: 17.

More preferably, the anti-CCR8-scFv region comprises any of the following sequences:

1) a light chain variable region having the amino acid sequence of SEQ ID NO: 12, and a heavy chain variable region having the amino acid sequence of SEQ ID NO: 13; or
2) a light chain variable region having the amino acid sequence of SEQ ID NO: 14, and a heavy chain variable region having the amino acid sequence of SEQ ID NO: 15.

The "extracellular region that binds to CCR8" may further comprise a spacer sequence at a C-terminus, and the anti-CCR8-scFv region and the "transmembrane region" may be linked via the spacer sequence. The spacer sequence region preferably comprises a Flag epitope-containing sequence region and/or a CD8A hinge region. Furthermore, the Flag epitope-containing sequence region preferably has the amino acid sequence of SEQ ID NO: 3, and the CD8A hinge region preferably has the amino acid sequence of SEQ ID NO: 4.

The "transmembrane region" means a region interposed between the extracellular region and the intracellular region. The transmembrane region is not particularly limited as long as it is a region interposed between the extracellular region and the intracellular region of a membrane protein, but a transmembrane region of a signaling molecule expressed in a lymphocyte that functions as an effector cell is preferred. Examples of the signaling molecule include CD8A, CD28, and NKG2D.

The "transmembrane region" preferably includes a CD8A transmembrane region or a CD28 transmembrane region. The CD8A transmembrane region preferably has the amino acid sequence of SEQ ID NO: 5, and the CD28 transmembrane region preferably has the amino acid sequence of SEQ ID NO: 6.

Here, when the "transmembrane region" comprises the CD28 transmembrane region, it is preferable that the intracellular region comprises a CD28 intracellular signal region at the same time. The CD28 intracellular signal region preferably has the amino acid sequence of SEQ ID NO: 7.

The "intracellular region" means a region that comprises an intracellular signal region of a signaling molecule expressed in a lymphocyte that functions as an effector cell, and that capable of transmitting a signal necessary for activation of the effector cell into the cell when the "extracellular region that binds to CCR8" binds to CCR8.

Examples of the "lymphocyte that functions as an effector cell" include an NK cell and a T cell, and an NK cell or a T cell is preferred. Examples of the signaling molecule include 4-1BB, 2B4, CD3£, DAP10, and DAP12.

The "intracellular region" preferably comprises at least one of intracellular signal regions of 4-1BB, 2B4, CD3£, and DAP10, more preferably comprises at least two thereof, further preferably comprises intracellular signal regions of 4-1BB and CD3£ or intracellular signal regions of 2B4 and CD3£, further preferably comprises intracellular signal regions of 4-1BB and CD3£, particularly preferably comprises a region in which a CD3£ intracellular signal region is linked to an end of a 4-1BB intracellular signal region, and most preferably comprises a region in which a 4-1BB intracellular signal region, a DAP10 intracellular signal region, and a CD3£ intracellular signal region are linked in this order.

When the "intracellular region" comprises a 4-1BB intracellular signal region, the 4-1BB intracellular signal region preferably has the amino acid sequence of SEQ ID NO: 8.

When the "intracellular region" comprises a 2B4 intracellular signal region, the 2B4 intracellular signal region preferably has the amino acid sequence of SEQ ID NO: 9.

When the "intracellular region" comprises a CD3£ intracellular signal region, the CD3£ intracellular signal region preferably has the amino acid sequence of SEQ ID NO: 10.

When the "intracellular region" comprises a DAP10 intracellular signal region, the DAP10 intracellular signal region preferably has the amino acid sequence of SEQ ID NO: 11.

Each region of the "extracellular region that binds to CCR8", the "linker sequence", the "spacer sequence region", the "transmembrane region", and the "intracellular region" can exhibit a similar effect as long as it has 90% or more identity, preferably 95% or more identity in amino acid sequence.

The chimeric antigen receptor of the present invention has cytotoxic activity against CCR8-expressing cells by being expressed in effector cells. The effector cell expressing the chimeric antigen receptor of the present invention can be produced by inserting cDNA encoding the chimeric antigen receptor of the present invention into an appropriate expression vector to construct a chimeric antigen receptor expression vector, and introducing the expression vector or mRNA of the chimeric antigen receptor of the present invention synthesized using the expression vector into an effector cell to express the chimeric antigen receptor.

The "cytotoxic activity" includes cell phagocytic activity by macrophages and the like.

Examples of the effector cell expressing the chimeric antigen receptor of the present invention include an NK cell, a T cell, and a macrophage, and an NK cell or a T cell is preferred, and an NK cell is more preferred. The effector cell can be obtained from peripheral blood, bone marrow, lymph node tissue, umbilical cord blood, thymus tissue, ascites, pleural effusion, spleen tissue, and the like collected from humans. The effector cell may be differentiated from a stem cell such as an iPS cell. Examples of the stem cell include a somatic stem cell such as a hematopoietic stem cell, an embryonic stem cell such as an ES cell, and a pluripotent stem cell such as an iPS cell, and an iPS cell is preferred. The effector cell may be used for autologous inoculation or for production of a cell preparation by allogeneic inoculation.

Examples of a medium for the effector cell expressing the chimeric antigen receptor of the present invention include a DMEM medium, an RPMI 1640 medium, an MEM medium, and a Ham's F-12 medium. In order to optimize culture conditions, various organic or inorganic substances including serum such as fetal bovine serum (FCS) and horse serum (HS), cytokines such as IL -2, amino acids, and hormones may be added to the medium.

Examples of the CCR8-expressing cell in which the effector cell expressing the chimeric antigen receptor of the present invention exhibits cytotoxic activity include a Treg cell and a tumor cell, and a Treg cell is preferred, and a tumor-infiltrating Treg cell is more preferred.

When the CCR8-expressing cell is a tumor-infiltrating Treg cell, the tumor is preferably a solid cancer. Specific examples of the solid cancer include breast cancer, uterine corpus cancer, cervical cancer, ovarian cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, bile duct carcinoma, gallbladder carcinoma, thyroid carcinoma, testicular cancer, thymic carcinoma, hepatocarcinoma, skin cancer, and brain tumor. Preferably, examples thereof include breast cancer, uterine corpus cancer, ovarian cancer, lung cancer, bladder cancer, colorectal cancer, kidney cancer, sarcoma, hepatocarcinoma, skin cancer, and brain tumor. When the CCR8-expressing cell is a tumor-infiltrating Treg cell, CCR8 may not be expressed in the tumor cell.

When the CCR8-expressing cell is a tumor cell, the tumor is preferably melanoma or hematological cancer. Examples of the hematological cancer expressing CCR8 include leukemias such as acute myeloid leukemia (AML) and acute lymphocytic leukemia (ALL), and lymphomas such as adult T-cell leukemia lymphoma (ATL), peripheral T-cell lymphoma (PTCL), cutaneous T-cell lymphoma (CTCL), diffuse large B-cell lymphoma (DLBCL), and Hodgkin lymphoma.

When the effector cell expressing the chimeric antigen receptor of the present invention exhibits cytotoxic activity, an amount of cytokine secreted in the effector cell increases, and the cytotoxic activity can be exhibited. Examples of the cytokine include IL-2 and interferon, and interferon-y is preferred.

The effector cell expressing the chimeric antigen receptor of the present invention is useful as a pharmaceutical composition. Therefore, a pharmaceutical composition containing the effector cell expressing the chimeric antigen receptor of the present invention can be administered systemically or locally. Examples of a route of administration include intravenous injection, such as infusion, intramuscular injection, intraperitoneal injection, and subcutaneous injection.

The pharmaceutical composition of the present invention may be a composition for autologous inoculation containing a cell obtained by expressing the chimeric antigen receptor of the present invention in an autologous effector cell, or may be a cell preparation by allogeneic inoculation containing a cell obtained by expressing the chimeric antigen receptor of the present invention in an allogeneic effector cell.

The pharmaceutical composition of the present invention is very useful as a medicament for the treatment and/or prevention of a CCR8-related disease. In particular, the pharmaceutical composition is very useful as a medicament for treating and/or preventing cancer in which intratumoral infiltration of CCR8-expressing Treg cells has occurred or cancer expressing CCR8. For example, the pharmaceutical composition of the present invention is very useful as a medicament for treating and/or preventing cancer such as breast cancer, uterine corpus cancer, cervical cancer, ovarian cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma, skin cancer, and brain tumor, preferably breast cancer, uterine corpus cancer, ovary cancer, lung cancer, bladder cancer, colorectal cancer, kidney cancer, sarcoma, hepatocarcinoma, skin cancer, and brain tumor. The pharmaceutical composition is also very useful as a medicament for treating and/or preventing leukemia and lymphoma.

The cancer according to the present invention includes all solid cancers and hematological cancers. Specific examples of the cancer include breast cancer, uterine corpus cancer, cervical cancer, ovarian cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma, skin cancer, and brain tumor. Preferably, examples thereof include breast cancer, uterine corpus cancer, ovarian cancer, lung cancer, bladder cancer, colorectal cancer, kidney cancer, sarcoma, hepatocarcinoma, skin cancer, and brain tumor. Leukemia and lymphoma are also preferred.

Note that the cancer according to the present invention shall mean not only epithelial malignancies such as ovarian cancer and stomach cancer, but also non-epithelial malignancies including hematopoietic cancer such as chronic lymphocytic leukemia and Hodgkin lymphoma. Furthermore, in the present description, the terms such as "cancer," "carcinoma," "tumor," and "neoplasm" are not distinguishable from one another and used interchangeably.

It is contemplated that a patient to be subjected to the pharmaceutical composition of the present invention is or is suspected of being a cancer patient. The pharmaceutical composition of the present invention contains a therapeutically effective amount of effector cells expressing the chimeric antigen receptor of the present invention, but contains, for example, $1 \times 10^4$ to $1 \times 10^9$ of the cells per administration. However, the pharmaceutical composition of the present invention is not limited to these doses. The administration period can also be appropriately selected depending on the age or symptoms of the patient. The pharmaceutical composition of the present invention may further comprise a pharmaceutically acceptable carrier or additive, depending on the route of administration. Examples of such carriers and additives include activating components of cells such as dimethyl sulfoxide (DMSO), serum albumin, various antibiotics, vitamins, cytokines, growth factors, and steroids, and surfactants acceptable as pharmaceutical additives. The additives used are selected as appropriate or in combination from the above, depending on the dosage form, but are not limited thereto.

The pharmaceutical composition of the present invention can be used alone or in combination with a composition or method for the treatment or prevention for the treatment or prevention of other cancers. Specifically, the composition or method of the present invention can be used in combination with a composition or method for chemotherapy or an immunological treatment method. Alternatively, physicochemical treatment methods such as radiation therapy, proton beam therapy, and hyperthermia can also be used in combination.

The present invention encompasses a polynucleotide encoding an amino acid sequence of the chimeric antigen receptor of the present invention. The present invention further encompasses an expression vector comprising the polynucleotide.

The polynucleotide is not particularly limited as long as it encodes the chimeric antigen receptor of the present invention, and it is a polymer consisting of nucleotides such as a plurality of deoxyribonucleic acids (DNAs) or ribonucleic acids (RNAs). It may contain a non-natural nucleotide. The polynucleotide of the present invention can be used for producing the chimeric antigen receptor of the present invention and the effector cell expressing the chimeric antigen receptor by a genetic engineering technique.

[EXAMPLES]

Hereinafter, the present invention will be explained in more detail by way of Examples of the present invention, but the present invention is not limited by them.

Test Example: Generation of anti-human CCR8 antibody

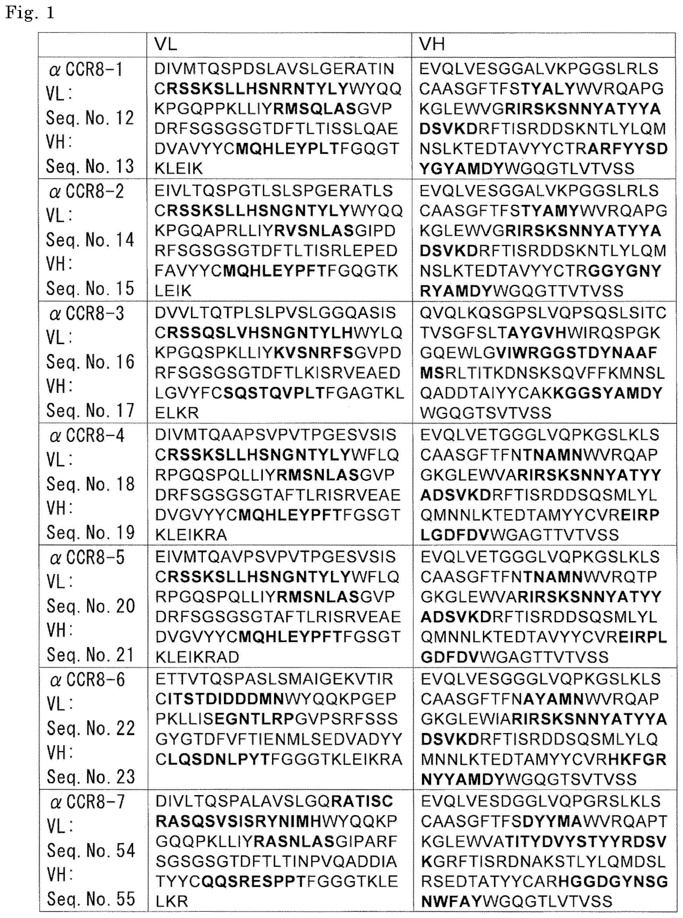

An anti-human CCR8 antibody was obtained based on the methods described in Examples 1 to 2, 4 to 7, and 9 of WO 2020/138489. The amino acid sequence of each variable region of the obtained antibody is shown in Figure 1, and SEQ ID NOs of the light chain variable region, the heavy chain variable region, and the CDRs are shown in Table 1.

Here, αCCR8-1 and αCCR8-2 are humanized monoclonal antibodies, αCCR8-3 to 6 are mouse-derived monoclonal antibodies, and αCCR8-7 is rat-derived monoclonal antibodies.

[Table 1]

| mAb | | | SEQ ID NO: | | SEQ ID NO: | | SEQ ID NO: | | SEQ ID NO: |
|---|---|---|---|---|---|---|---|---|---|
| αCCR8-1 | Light chain variable region | | 12 | CDR1 | 24 | Heavy chain variable region | 13 | CDR1 | 27 |
| | | | | CDR2 | 25 | | | CDR2 | 28 |
| | | | | CDR3 | 26 | | | CDR3 | 29 |
| αCCR8-2 | Light chain variable region | | 14 | CDR1 | 30 | Heavy chain variable region | 15 | CDR1 | 33 |
| | | | | CDR2 | 31 | | | CDR2 | 28 |
| | | | | CDR3 | 32 | | | CDR3 | 34 |

(continued)

| mAb | | SEQ ID NO: | | SEQ ID NO: | | SEQ ID NO: | | SEQ ID NO: |
|---|---|---|---|---|---|---|---|---|
| αCCR8-3 | Light chain variable region | 16 | CDR1 | 35 | Heavy chain variable region | 17 | CDR1 | 38 |
| | | | CDR2 | 36 | | | CDR2 | 39 |
| | | | CDR3 | 37 | | | CDR3 | 40 |
| αCCR8-4 | Light chain variable region | 18 | CDR1 | 30 | Heavy chain variable region | 19 | CDR1 | 42 |
| | | | CDR2 | 41 | | | CDR2 | 28 |
| | | | CDR3 | 32 | | | CDR3 | 43 |
| αCCR8-5 | Light chain variable region | 20 | CDR1 | 30 | Heavy chain variable region | 21 | CDR1 | 42 |
| | | | CDR2 | 41 | | | CDR2 | 28 |
| | | | CDR3 | 32 | | | CDR3 | 43 |
| αCCR8-6 | Light chain variable region | 22 | CDR1 | 44 | Heavy chain variable region | 23 | CDR1 | 47 |
| | | | CDR2 | 45 | | | CDR2 | 28 |
| | | | CDR3 | 46 | | | CDR3 | 48 |
| αCCR8-7 | Light chain variable region | 54 | CDR1 | 56 | Heavy chain variable region | 55 | CDR1 | 59 |
| | | | CDR2 | 57 | | | CDR2 | 60 |
| | | | CDR3 | 58 | | | CDR3 | 61 |

Example 1: mRNA synthesis of chimeric antigen receptor that recognizes CCR8 as antigen (anti-CCR8-CAR)

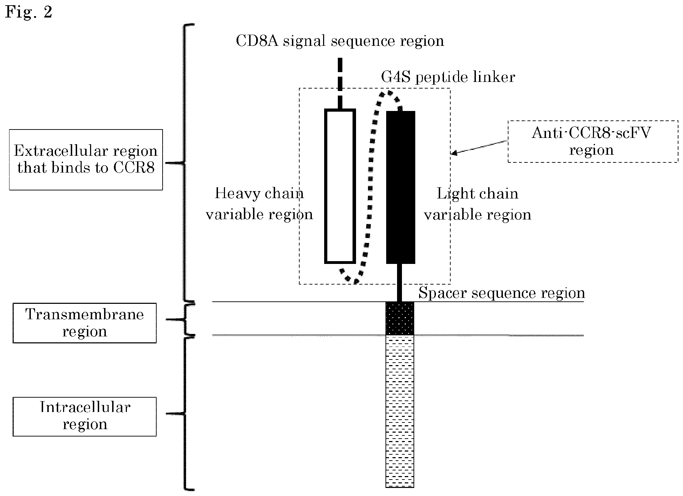

An anti-CCR8-CAR-carrying vector was constructed based on the amino acid sequences of the light chain variable region and the heavy chain variable region in the anti-human CCR8 antibody obtained in Test Example. A structure of the anti-CCR8-CAR is shown in Figure 2.

As an extracellular region that binds to CCR8, a CD8A signal sequence region (SEQ ID NO: 1), an anti-CCR8-scFv region (an anti-human CCR8 antibody heavy chain variable region, a G4S peptide linker (SEQ ID NO: 2), and an anti-human CCR8 antibody light chain variable region were linked in this order), and a spacer sequence region (a Flag epitope-containing sequence region (SEQ ID NO: 3) and a CD8A hinge region (SEQ ID NO: 4) were linked in this order) were linked in this order from an N-terminal side.

A transmembrane region was linked to a C-terminus side of the above extracellular region that binds to CCR8. As the transmembrane region, a CD8A transmembrane region (SEQ ID NO: 5) or a CD28 transmembrane region (SEQ ID NO: 6) was used.

An intracellular region was linked to a C-terminus side of the above transmembrane region. As the intracellular region, a plurality of intracellular signal regions among a 4-1BB intracellular signal region (SEQ ID NO: 8), a 2B4 intracellular signal region (SEQ ID NO: 9), a CD3£ intracellular signal region (SEQ ID NO: 10), and a DAP10 intracellular signal region (SEQ ID NO: 11) were linked and used.

When the transmembrane region contained the CD28 transmembrane region, a CD28 intracellular signal region (SEQ ID NO: 7) was simultaneously inserted into an N-terminal side of the intracellular region.

DNA encoding the above anti-CCR8-CAR was generated by custom synthesis at Fasmac Co., Ltd. The DNA was cloned into an SmaI site of a pUC19 vector in a direction in which an EcoRI site was upstream of the gene. Using the cloning vector as a template, the anti-CCR8-CAR gene region was amplified by a PCR method using a pUC19-T7 primer (SEQ ID NO: 50) and a pUC19-R1 primer (SEQ ID NO: 51). As PCR conditions, 10 ng of template DNA was used, and a target region was amplified by performing 35 cycles of 94°C → 65°C → 72°C using a KOD polymerase. A DNA amount of the amplified anti-CCR8-CAR gene was quantified, mRNA was synthesized using iScribe T7 ARCA mRNA kit (manufactured by NEW ENGLAND BioLabs) according to the method described in the kit, and polyA was added to obtain anti-CCR8-CAR mRNA.

As a negative control, an opposite strand of an ampicillin gene was amplified by PCR using a pUC19 plasmid as a template using the following primers (T7-AMP-F2 primer (SEQ ID NO: 52) and AMP-R1 primer (SEQ ID NO: 53)). mRNA was synthesized from the amplified DNA in the same manner as described above, and polyA was added to obtain negative control mRNA.

Example 2: Culture of human NK cell line KHYG-1 cells and introduction of mRNA

KHYG-1 cells, a human NK cell line, were cultured at 37°C under 5% $CO_2$ in the presence of antibiotics using RPMI 1640/10% FCS to which human IL-2 (PeproTech, Inc.) was added to a final concentration of 100 units/ml. When the cell concentration reached 2 to 3 $\times$ $10^5$/ml, the cells were used for mRNA introduction.

The KHYG-1 cells were washed twice with OPTI-MEM and suspended to 2.5 $\times$ $10^6$ cells/100 $\mu$l. 3 $\mu$l (6 pg) of each mRNA was added to a NEPA21 dedicated cuvette (2 mm gap) containing 100 $\mu$l of the cell solution, and NEPA21 electroporation (Nepa Gene Co., Ltd.) was performed under the following conditions. The introduction conditions followed the method described in the protocol. The cells after mRNA introduction were cultured at 37°C under 5% $CO_2$ for 16 to 18 hours.

The transformed cells were used for evaluation of cytotoxic activity against human CCR8-expressing cells as target cells within 20 hours after mRNA introduction.

Example 3: Preparation of human CCR8-expressing cells

(1) Production of Rat-1 cells and RAMOS cells expressing human CCR8

Human CCR8 (SEQ ID NO: 49) was cloned into a pQCXIP (alternatively, a vector obtained by converting a CMV promoter of a pQEF or pQCXIP vector into a human EF promoter) vector by a conventional method, and the expressed human CCR8 expression vector was transformed into Rat-1 cells (pQCXIP vector) and RAMOS cells (pQEF vector) by a conventional method using Lipofectamine 3000. After 2 days, puromycin was added at 1 pg/ml, and the transformed cells were selected. A human CCR8 expression rate of the cells was confirmed using a PE-labeled anti-human CCR8 antibody (clone name: L263G8, BioLegend, Inc.) and using a flow cytometer.

In the case of the RAMOS cells, cell cloning was performed so that the cells further become a single cell after drug selection. Expression of human CCR8 was confirmed using a PE-labeled anti-human CCR8 antibody and using a flow cytometer in the same manner as described above. In addition, the number of molecules expressing human CCR8 per cell was determined according to the method described in the kit using QIFIKIT (DAKO).

(2) Labeling human CCR8-expressing cells with calcein

Human CCR8-expressing cells to be labeled were suspended in a DMEM/10% FCS medium so as to be 5 $\times$ $10^5$ cells/ml, and calcein (Calcein-AM, 349-07201, DOJINDO LABORATORIES) dissolved in DMSO was added so as to have a final concentration of 4 pg/ml. The final concentration of DMSO was adjusted to 8% or less. The cell culture solution was suspended, and then cultured at 37°C under 5% $CO_2$ for 30 minutes. Thereafter, the cells were washed twice with a buffer for cytotoxic activity evaluation (DMEM/4% FCS) and used for cytotoxic activity evaluation.

Example 4: Cytotoxic activity of human CCR8-expressing cells by anti-CCR8-CAR-expressing KHYG-1 cells

mRNA of an anti-CCR8-CAR in which an extracellular region that binds to CCR8 including a variable region of αCCR8-1 or αCCR8-2 among the anti-CCR8 antibodies, a CD8A transmembrane region, and an intracellular region in which a 2B4 intracellular signal region and a CD3£ intracellular signal region were linked were linked, generated by the method of Example 1 (referred to as αCCR8-1-CAR or αCCR8-2-CAR, respectively), was introduced into KHYG-1 cells. As a control, the negative control mRNA generated by the method of Example 1 was introduced.

20 hours after transformation of the KHYG-1 cells, the transformed KHYG-1 cells were mixed with calcein-labeled human CCR8-expressing Rat-1 cells generated in Example 3 as target cells or Rat-1 cells as parent cells in 160 $\mu$l of a buffer for cytotoxic activity evaluation so that a ratio of the transformed KHYG-1 cells to the target cells (E/T cell ratio) was 3 : 1.

Cytotoxic activity was evaluated using 96-well round-bottom dishes. After mixing the transformed KHYG-1 cells and the target cells, they were centrifuged using a plate centrifuge at 1000 rpm for 2 minutes to allow the cells to settle. The cells were mixed, and then cultured at 37°C under 5% $CO_2$ for 2 hours. Thereafter, 60 $\mu$l of the cell supernatant was slowly separated from the plate. For the separated supernatant, an amount of fluorescence contained in the supernatant was quantified using a fluorescence measuring instrument within 30 minutes.

A spontaneous release amount (BG) of calcein from each target cell was determined by measuring the supernatant of the well of each target cell only in the same manner. A total calcein release amount (TOTAL) was determined by adding 1% Triton X-100 to the well of each target cell only so that Triton X-100 was 0.1% and measuring the same amount of supernatant.

As a method for calculating cytotoxic activity by using an amount of calcein released from a killed target cell as an index, calculation was performed by the following formula.

$$\{(\text{Fluorescence value of each well} - \text{BG fluorescence value})/((\text{TOTAL fluorescence value} - \text{BG fluorescence value})\} \times 100\ (\%)$$

Spontaneous release was subtracted, and assuming that an amount of fluorescence in the supernatant when all cells were killed (in this case, an amount of fluorescence released to the supernatant by complete cell lysis by Triton) was 100%, a killing rate (%) was calculated.

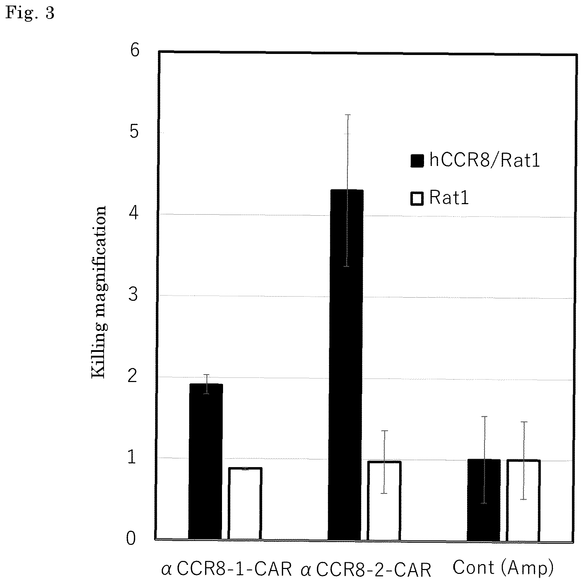

The results are shown in Figure 3. Assuming that a killing rate was 1 when the negative control mRNA-introduced KHYG-1 cells were mixed with the Rat-1 cells as parent cells and the human CCR8-expressing Rat-1 cells, a killing rate of the αCCR8-1-CAR-expressing KHYG-1 cells and the αCCR8-2-CAR-expressing KHYG-1 cells was shown at a killing magnification relative to the killing rate of 1. As compared to the case where the negative control mRNA was transformed, in the cells transformed with the αCCR8-1 CAR or the αCCR8-2-CAR, human CCR8-expressing cell-dependent and anti-CCR8-CAR-expressing cell-specific cytotoxic activity was observed. On the other hand, no specific cytotoxic activity by the anti-CCR8-CAR was observed in the parent cells not expressing human CCR8 (Rat-1).

Example 5: Examination of transmembrane region and intracellular region (1)

mRNA of each anti-CCR8-CAR in Table 2 generated by the method in Example 1 was introduced into KHYG-1 cells. As a control, the negative control mRNA generated by the method of Example 1 was introduced.

18 hours after transformation of KHYG-1 cells, an expression rate of each anti-CCR8-CAR on a cell surface was quantified using an anti-FLAG antibody (PE-anti-DYKDDDDK Tag Clone: 1.5, BioLegend, Inc.). The cells were stained with LIVE/DEAD (Thermo Fisher Scientific Inc.) and the anti-FLAG antibody at room temperature for 1 hour, washed once with a buffer for cytotoxic activity evaluation, and then the cell surface expression rate of the anti-CCR8-CAR was evaluated by a flow cytometer. As an analysis method, live cells were gated by LIVE/DEAD, and then major cell population regions were gated by FSC/SSC, and an expression level of FLAG was analyzed for this cell population. A threshold value was set at a value at which a FLAG-positive rate was about 1 to 2% in control (AMP) transformed cells, and a cell ratio of about 1 to 2% or more was calculated as the expression rate. Next, in order to equalize the expression rate of each anti-CCR8-CAR, the expression rate was adjusted to an expression rate of a cell having the lowest expression rate. Specifically, the control transformed cells and the anti-CCR8-CAR transformed cells were mixed at a certain ratio, and the expression rate of the anti-CCR8-CAR was adjusted to about 30%. The expression rate of the anti-CCR8-CAR in any cell is around 30%. Cytotoxic activity was evaluated using the adjusted KHYG-1 cells.

The transformed KHYG-1 cells and calcein-labeled human CCR8-expressing Rat-1 cells were mixed under conditions where the E/T cell ratio was 10 : 1, 3 : 1, and 1 : 1, and calcein release was evaluated. Calcein release was evaluated in the same manner as in Example 4. When an actual number of cells is 10 : 1, the number of effector cells is $3 \times 10^4$ and the number of target cells is $3 \times 10^3$ in 150 µl of the buffer for cytotoxic activity evaluation.

[Table 2]

| | Anti-CCR8 antibody | Transmembrane region | Intracellular region |
|---|---|---|---|
| αCCR8-2-CAR-1 | αCCR8-2 | CD8A | 2B4-CD3ζ |
| αCCR8-2-CAR-2 | αCCR8-2 | CD8A | 4-1BB-CD3ζ |
| αCCR8-2-CAR-3 | αCCR8-2 | CD8A | CD3ζ-DAP10 |
| αCCR8-2-CAR-4 | αCCR8-2 | CD28 | CD28-2B4-CD3ζ |
| αCCR8-2-CAR-5 | αCCR8-2 | CD28 | CD28-4-1BB-CD3ζ |
| αCCR8-2-CAR-6 | αCCR8-2 | CD28 | CD28-CD3ζ-DAP10 |

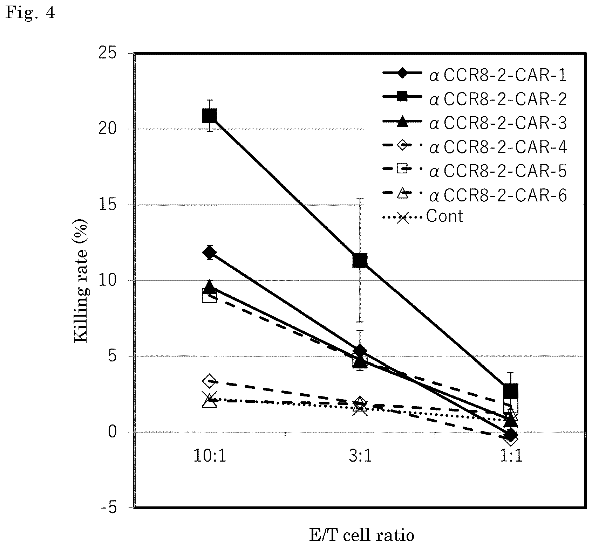

The results are shown in Figure 4. The anti-CCR8-CAR with the highest cytotoxic activity was αCCR8-2-CAR-2. A significant difference test was performed between αCCR8-2-CAR--2 and the other anti-CCR8-CARs. A Tukey's multiple comparison test was performed and there were significant differences at $P < 0.05$ for all items for αCCR8-2-CAR-2 and the other anti-CCR8-CARs.

For a combination in which only the transmembrane region (CD8A or CD28) was changed and the other regions were exactly the same in this experiment, cytotoxic activity in KHYG-1 cells was found to be excellent in the sequence of the transmembrane region of CD8A in all anti-CCR8-CARs.

In a comparison between the anti-CCR8-CARs of the same transmembrane region, a chimeric receptor having an intracellular signal region of 4-1BB in both cases of CD8A and CD28 as the transmembrane region had the highest activity, and thus it was found that a combination of intracellular signal regions of 4-1BB and CD3£ was excellent in KHYG-1 cells.

From the above, it was found that in NK cells expressing an anti-CCR8-CAR, a combination of a transmembrane region of CD8A and an intracellular signal region of 4-1BB-CD3ζ has the strongest cytotoxic activity.

Example 6: Examination of transmembrane region and intracellular region (2)

Cytotoxic activity of each anti-CCR8-CAR-expressing KHYG-1 cell of Table 3 was evaluated in the same manner as in Example 5. The evaluation was performed under conditions where the E/T cell ratio was 27 : 1 and 3 : 1.

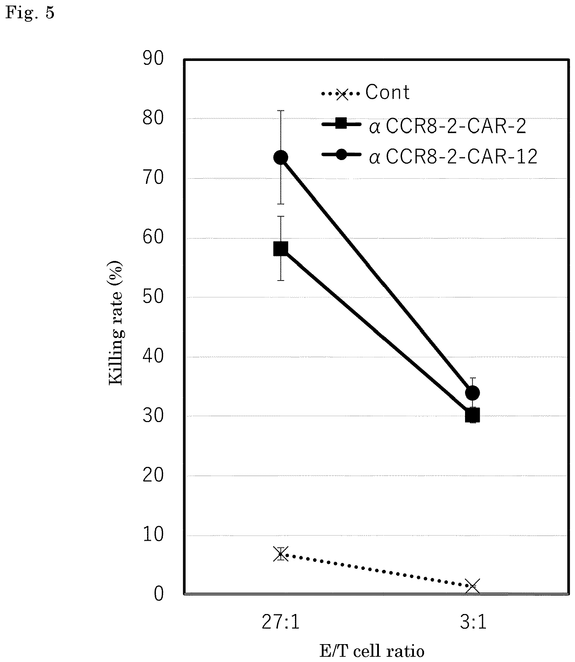

The results are shown in Figure 5. When the E/T cell ratio was 27 : 1, αCCR8-2-CAR-12-expressing KHYG-1 cells were found to have significantly increased killing activity by about 1.2 fold compared to αCCR8-2-CAR-2-expressing KHYG-1 cells (P < 0.05 by Tukey's multiple comparison test).

From the above results, it was found that by adding DAP10 to the intracellular region of αCCR8-2-CAR-2, stronger cytotoxic activity is obtained than that of αCCR8-2-CAR-2-expressing KHYG-1 cells.

[Table 3]

| | Anti-CCR8 antibody | Transmembrane region | Intracellular region |
|---|---|---|---|
| αCCR8-2-CAR-2 | αCCR8-2 | CD8A | 4-1BB-CD3ζ |
| αCCR8-2-CAR-12 | αCCR8-2 | CD8A | 4-1BB-DAP10-CD3ζ |

Example 7: Examination of transmembrane region and intracellular region (3)

Cytotoxic activity of each anti-CCR8-CAR-expressing KHYG-1 cell of Table 4 was evaluated in the same manner as in Example 5. The expression rate of the anti-CCR8-CAR was adjusted to about 40%, and the evaluation was performed under conditions where the E/T cell ratio was 10 : 1, 3 : 1, and 1 : 1.

The results are shown in Figure 6A. When the E/T cell ratio was 10 : 1 and 3 : 1, Tukey's multiple comparison test was performed, and there were significant differences at P < 0.05 for both E/T cell ratios between αCCR8-2-CAR-12 and the other anti-CCR8-CARs. The anti-CCR8-CAR having the most potent killing activity among the three was found to be αCCR8-2-CAR-12.

Furthermore, when the same experiment for evaluating cytotoxic activity was performed for 26 hours, and an amount of interferon-y secreted into the culture supernatant after 26 hours was measured by an IFN-y ELISA kit (BPS Bioscience, Inc.), a significant increase in secretion of interferon-y was observed in the culture supernatant of each anti-CCR8-CAR-expressing KHYG-1 cell in Table 4 (Figure 6B, Student t-test, P < 0.001).

[Table 4]

| | Anti-CCR8 antibody | Transmembrane region | Intracellular region |
|---|---|---|---|
| αCCR8-2-CAR-11 | αCCR8-2 | CD8A | 4-1BB-2B4-CD3ζ |
| αCCR8-2-CAR-12 | αCCR8-2 | CD8A | 4-1BB-DAP10-CD3ζ |
| αCCR8-2-CAR-13 | αCCR8-2 | CD8A | 4-1BB-2B4-DAP10-CD3ζ |

Example 8: Examination of anti-CCR8-scFv region (1)

The anti-CCR8-scFv region was examined in the same manner as in Example 5. Six kinds of anti-CCR8-CARs shown in Table 5, which differ only in the antigen-binding region of the extracellular region from the amino acid sequence of the variable region of the anti-human CCR8 antibody, were designed. The sequences were all the same except for those of the antibody region, a CD8A transmembrane region was used as the transmembrane region, and a 2B4-CD3ζ intracellular signal region was used as the intracellular region for all anti-CCR8-CARs. The expression rate of the anti-CCR8-CAR was adjusted to about 36%, and the evaluation was performed under conditions where the E/T cell ratio was 10 : 1, 3 : 1, and 1 : 1.

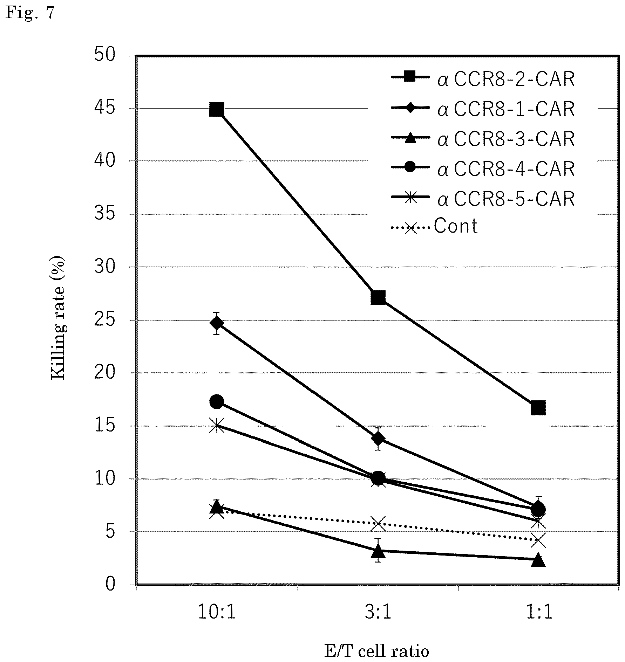

The results are shown in Figure 7. αCCR8-2-CAR-expressing KHYG-1 cells had the strongest cytotoxic activity, followed by αCCR8-1-CAR-expressing KHYG-1 cells. By Tukey's multiple test, the αCCR8-2-CAR-expressing KHYG-1 cells were significantly different for all the other anti-CCR8-CAR-expressing KHYG-1 cells. αCCR8-1-CAR-expressing KHYG-1 cells were significantly higher for all anti-CCR8-CAR-expressing KHYG-1 cells other than the αCCR8-2-CAR-expressing KHYG-1 cells under conditions where the E/T cell ratio was 10 : 1 and 3 : 1.

[Table 5]

|  | Anti-CCR8 antibody | Transmembrane region | Intracellular region |
|---|---|---|---|
| αCCR8-1-CAR | αCCR8-1 | CD8A | 2B4-CD3ζ |
| αCCR8-2-CAR | αCCR8-2 | CD8A | 2B4-CD3ζ |
| αCCR8-3-CAR | αCCR8-3 | CD8A | 2B4-CD3ζ |
| αCCR8-4-CAR | αCCR8-4 | CD8A | 2B4-CD3ζ |
| αCCR8-5-CAR | αCCR8-5 | CD8A | 2B4-CD3ζ |
| αCCR8-6-CAR | αCCR8-6 | CD8A | 2B4-CD3ζ |

Example 8-2: Examination of anti-CCR8-scFv region (2)

mRNA of an anti-CCR8-CAR in which an extracellular region that binds to CCR8 including a variable region of αCCR8-7 among the anti-CCR8 antibodies, a CD8A transmembrane region, and an intracellular region in which a 2B4 intracellular signal region and a CD3£ intracellular signal region were linked were linked, generated by the method of Example 1 (referred to as αCCR8-7-CAR), was introduced into KHYG-1 cells. As a control, the negative control mRNA generated by the method of Example 1 was introduced.

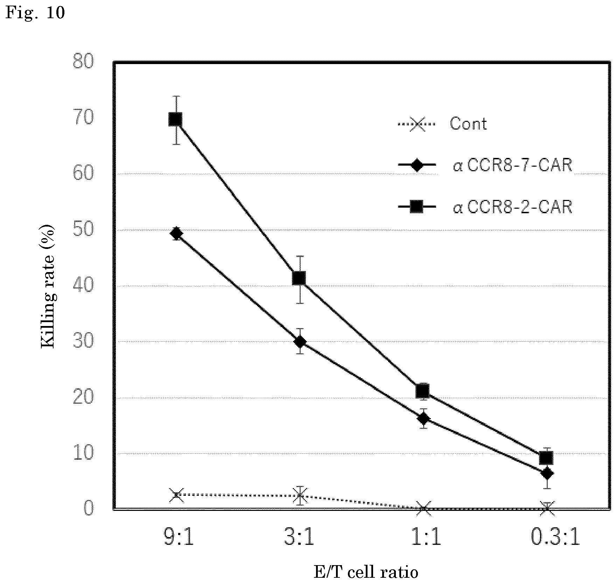

Cytotoxic activity of αCCR8-7-CAR-expressing KHYG-1 cells was evaluated in the same manner as in the above (1) (Figure 10). The evaluation was performed under conditions where the E/T cell ratio was 9 : 1, 3 : 1, 1 : 1, and 0.3 : 1.

Example 9: Evaluation of cytotoxic activity of anti-CCR8-CAR-expressing KHYG-1 cells against human CCR8-expressing RAMOS cells expressing CCR8 to the same extent as CCR8-expressing tumor-infiltrating Treg cells

For the human CCR8-expressing RAMOS cells (C5 cells and C6 cells) generated in Example 3 and RAMOS cells as parent cells, an expression level of human CCR8 was confirmed by the method in Example 3 using a flow cytometer. Furthermore, the approximate average number of human CCR8-expressing molecules on a cell surface per cell was determined according to the protocol using QIFIKIT (DAKO) and an anti-human CCR8 antibody (unlabeled L263G8, BioLegend, Inc.).

As a result, the number was 2630 molecules/cell for the C5 cells and 5950 molecules/cell for the C6 cells. In addition, the average number of CCR8-expressing molecules on a cell surface of CCR8-expressing tumor-infiltrating Treg cells of human lung cancer was evaluated in the same manner, and was about 4000 molecules/cell, and thus it could be confirmed that the C5 cells and the C6 cells expressed CCR8 to the same extent as the CCR8-expressing tumor-infiltrating Treg cells.

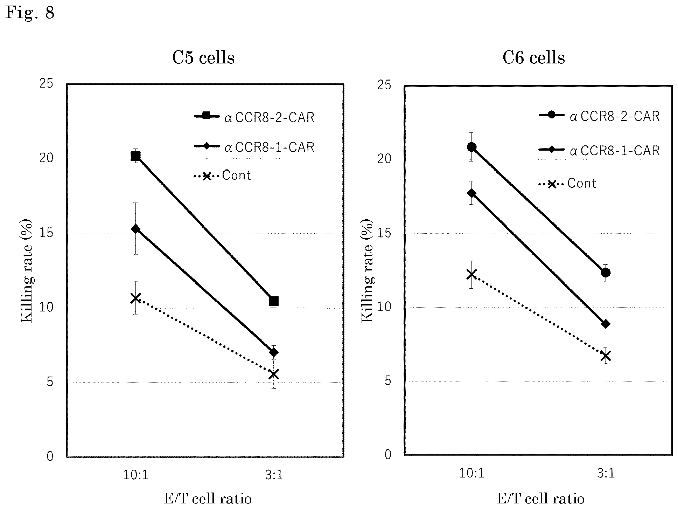

The C5 cells and the C6 cells, which are human CCR8-expressing RAMOS cells, were used as target cells, and cytotoxic activity of αCCR8-1-CAR-expressing KHYG-1 cells and αCCR8-2-CAR-expressing KHYG-1 cells was evaluated in the same manner as in Example 5 (Figure 8). The evaluation was performed under conditions where the E/T cell ratio was 10 : 1 and 3 : 1.

As a result, in both C5 and C6 cells, at both E/T cell ratios of 10 : 1 and 3 : 1, the αCCR8-2-CAR-expressing KHYG-1 cells had significantly increased cytotoxic activity compared to the αCCR8-1-CAR-expressing KHYG-1 cells and the negative control (P < 0.05 for both pairs by Tukey's multiple comparison test). For the αCCR8-1-CAR-expressing KHYG-1 cells, both C5 and C6 cells had significantly increased cytotoxic activity compared to the negative control only when the E/T cell ratio was 10 : 1 (P < 0.05 for both cells by Tukey's multiple comparison test).

From the above, it was found that the αCCR8-1-CAR-expressing KHYG-1 cells and the αCCR8-2-CAR-expressing KHYG-1 cells retain sufficient cytotoxic activity even against cells having an expression level equivalent to or of about 50% of an expression level of CCR8 expressed on the cell surface of the CCR8-expressing tumor-infiltrating Treg cells.

Example 10: Evaluation of cytotoxic activity of anti-CCR8-CAR-expressing KHYG-1 cells against human Treg cells

(1) Preparation of human Treg cells

PBMCs were separated from peripheral blood using Ficoll-Paque (Ficoll-Paque PLUS, GEW Healthcare). CD4+ T cells were purified therefrom using CD4 + T Cell Isolation Kit human (Miltenyi Biotec). Thereafter, using a FITC-labeled anti-human CD4 antibody, a PerCP_Cy5.5-labeled anti-human CD45RA antibody, a PECy7-labeled anti-human CD25 antibody, and a Bv510-labeled anti-human CD127 antibody, CD45RA+CD127-CD4+CD25+ cells were sorted (FACS Aria, Becton, Dickinson and Company) by a sorting function of FACS_Aria. Human Treg cells were separated by this sorting.

To the separated Treg cells, Dynabeads Human Treg Expander (VERITAS Corporation) was added according to the protocol, and cultured for 20 days at 37°C under 5% $CO_2$ using an RPMI 1640/10% FCS medium by a method according to the protocol. As a result of detecting an expression rate of a human CCR8 protein on a cell surface of the cultured Treg cells by a flow cytometer by the method of Example 3, the positive rate was found to be about 20%.

(2) Cytotoxic activity of anti-CCR8-CAR-expressing KHYG-1 cells against human Treg cells

Using the Treg cells obtained in (1) as target cells, KHYG-1 cells transformed with αCCR8-2-CAR mRNA or negative control mRNA were used as effector cells. Using an anti-FLAG antibody in the same manner as in Example 5, an αCCR8-2-CAR expression rate on a KHYG-1 cell surface at 24 hours after transformation was calculated to be 70%.

Cytotoxic activity evaluation was started using 96-well round-bottom plates at 7 hours after transformation and performed for 16 hours. Cytotoxic activity evaluation was started by mixing cells at E/T cell ratios of 10 : 1, 3 : 1, and 1 : 1. For the number of each cell in 96 wells, the number was $1 \times 10^4$ cells/well for all the target Treg cells. The number of the transformed KHYG-1 cells was $1 \times 10^5$ to $1 \times 10^4$ according to the E/T cell ratio. RPMI 1640/10% FCS was used as a culture solution at the time of activity evaluation.

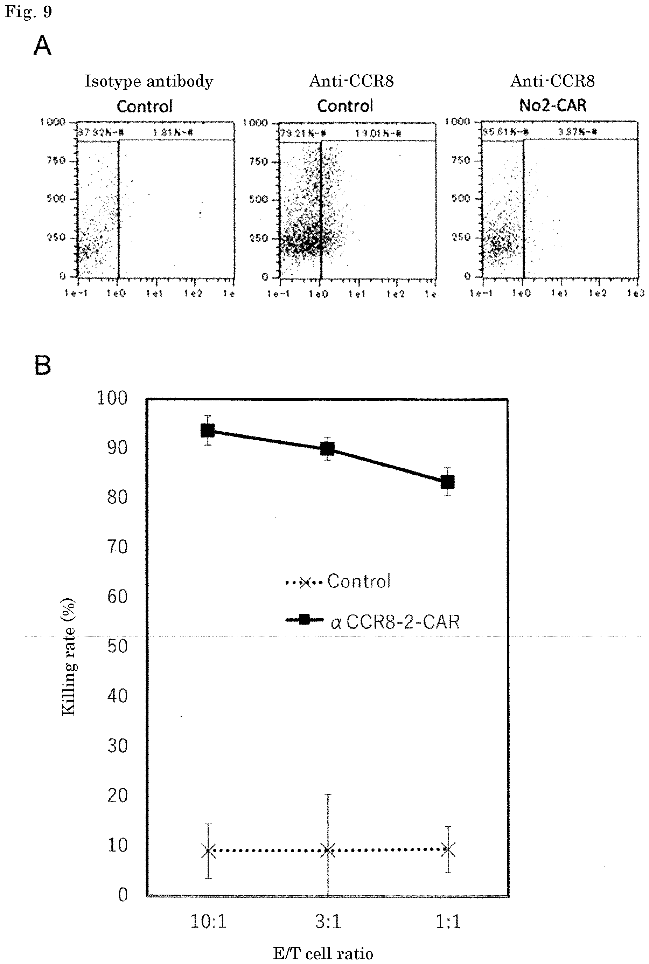

After 16 hours from the start of the evaluation, in order to block an Fc receptor, an amount of 1/20 of Human TruStain FcX (BioLegend, Inc.) was added to each well, and the mixture was allowed to stand at room temperature for 15 minutes. Thereafter, the cells were washed once with an HBSS/2% FCS/10 mM HEPES buffer, suspended in 100 μl of the same buffer, and then stained at 4°C for 1 hour using LIVE/DEAD (Thermo Fisher Scientific Inc.), Bv421-labeled anti-human CD4 (BD Biosciences), PE-labeled anti-human CCR8 (BioLegend, Inc.), or a PE-labeled isotype control antibody (BioLegend, Inc.). As cells used for isotype antibody staining, cells obtained by mixing the Treg cells and the transformed KHYG-1 cells at an E/T cell ratio of 3 : 1 were used. In addition, all of the figures in which cytotoxic activity was evaluated in Figure 9A are examples of cells mixed at 10 : 1. After 16 hours of culture, the cells were washed twice with the same buffer, and a percentage of surviving human CCR8-expressing cells was calculated by a flow cytometer for analysis. For identification of human CCR8-expressing cells, a background threshold value was set with an isotype control antibody, and cells having a fluorescence intensity higher than the threshold value in anti-CCR8 staining were used as expressing cells. In identification of the surviving human CCR8-expressing cells, CD4-expressing cells were gated, and negative fractions by LIVE/DEAD staining were gated, among which CCR8-positive fractions were regarded as the surviving human CCR8-expressing cells. A percentage of human CCR8-expressing cells in the wells containing only the target cells was calculated as 100%. Figure 9A shows examples of the analysis with a flow cytometer.

As a result, as shown in Figure 9B, in the cells transformed with the negative control mRNA, the killing rate was about 15% at any E/T cell ratio, but in the KHYG-1 cells transformed with the αCCR8-2-CAR, the killing rate was 80% or more at all E/T cell ratios, and a significant increase in the killing rate was observed as compared to Control. (P < 0.001 for all E/T cell ratios in Student t-test).

From the above, it was confirmed that NK cells expressing αCCR8-2-CAR have cytotoxic activity against Treg cells expressing human CCR8.

Example 11: Cytotoxic activity evaluation using peripheral blood-derived NK cells expressing anti-CCR8-CAR

(1) Identification of cell lines expressing human CCR8

ATL patient-derived cell line ATN-1 cells and ALL patient-derived cell lines TALL-1 cells and CCRF-HSB2 cells (all RIKEN) were stained using a PE-labeled anti-human CCR8 antibody (PE-labeled L263G8, BioLegend, Inc.) at a final concentration of 4 pg/ml according to the protocol in the same manner as in Example 9. A positive signal was detected for both cells by flow cytometer analysis. Regarding this positive signal, when an αCCR8-1 antibody, which is an anti-CCR8 antibody shown in Table 1, was added at a concentration of 250 times (1 mg/ml) 5 minutes before the start of staining, the positive signal completely disappeared.

From the above, it was confirmed that the positive signal of these cells was expression of human CCR8, and it was found that human CCR8 was expressed in these cells.

(2) Cytotoxic activity evaluation using peripheral blood-derived NK cells

Human peripheral blood-derived NK cells (Biotherapy Institute of Japan, Inc.) were cultured and expanded in RPMI 1640/10% FCS/Nonessential amino acid + human IL-2 (200 U/ml) (PeproTech, Inc.) for 2 weeks. As a result of staining a CD16-positive rate after culture with a PE-anti-human CD16 antibody (3G8, BioLegend, Inc.) and a FITC-anti-human CD56 antibody (HCD56, BioLegend, Inc.), a proportion of cells positive for both markers was 95% or more as compared to the case of isotype antibody staining (MOPC21, BioLegend, Inc.). According to Examples 2 and 4, these cells were transformed with 5 pg of αCCR8-2-CAR-2 mRNA using NEPA21. The CAR-positive rate 16 hours after transformation was 10.6%. Using these cells, cytotoxic activity was evaluated by a calcein method using human CCR8-expressing Rat-1 cells as target cells.

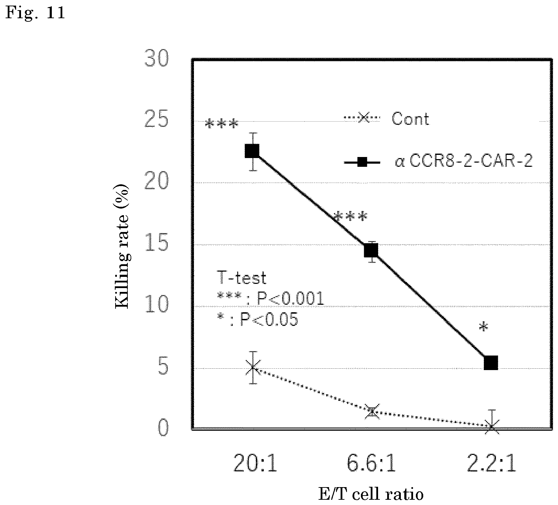

As a result, as compared to untreated peripheral blood-derived NK cells as parent cells and Amp mRNA-introduced peripheral blood-derived NK cells as a control, peripheral blood-derived NK cells expressing αCCR8-2-CAR-2 significantly increased cytotoxic activity against human CCR8-expressing Rat-1 cells (Figure 11).

From the above, it was found that by expressing αCCR8-2-CAR-2 mRNA in peripheral blood-derived NK cells, cytotoxic activity is exhibited against human CCR8-expressing Rat-1 cells.

Next, cytotoxic activity was evaluated in the same manner against ALL cell line TALL-1 cells. As a result, similarly to human CCR8-expressing Rat-1 cells, it was found that ALL cell line TALL-1 cells can be killed by expressing αCCR8-2-CAR-2 mRNA in peripheral blood-derived NK cells.

Example 12: Cytotoxic activity evaluation using peripheral blood-derived T cells expressing anti-CCR8-CAR

(1) Separation of T cells from human peripheral blood

Peripheral blood was separated from a healthy subject, and T cells were separated using a human CD8-positive T cell isolation kit (MojoSort, BioLegend, Inc.). These cells were cultured for 2 weeks in the presence of RPMI 1640/10% FCS/Nonessential amino acid + Dynabeads human T-Activator CD3/CD28 (Gibco) + human IL-2 (200 U/ml) (PeproTech, Inc.). Each T cell subset of a T cell population after culturing for 2 weeks was evaluated by a flow cytometer using various antibodies. As detection antibodies, PE-CD16 (B73.1, BioLegend, Inc.), APC-CD8 (SK1, BioLegend, Inc.), FITC-CD4 (OKT4, BioLegend, Inc.), Bv421-CD3 (SK7, BioLegend, Inc.), LIVE/DEAD (APC-Cy7), and an isotype antibody (MOPC-21, BioLegend, Inc.) were used.

As a result, proportions of CD3-positive, CD4-positive, CD8-positive, and CD16-positive cells were 99.6%, 51.6%, 41.6%, and 0.5%, respectively, most of them were CD3-positive T cells, and CD16-positive NK cells were hardly observed.

(2) Cytotoxic activity evaluation using peripheral blood-derived T cells

According to Examples 2 and 4, these cells were transformed with 5 pg of αCCR8-2-CAR-2 or αCCR8-2-CAR-12 mRNA using NEPA21. According to Examples 2 and 4, 16 hours after transformation, cytotoxic activity was evaluated using ATN-1 cells, which are a calcein-labeled ATL cell line, as target cells. The CAR-positive rates of αCCR8-2-CAR-2 and αCCR8-2-CAR-12 in peripheral blood-derived T cells 16 hours after transformation were 5.9% and 3.3%, respectively.

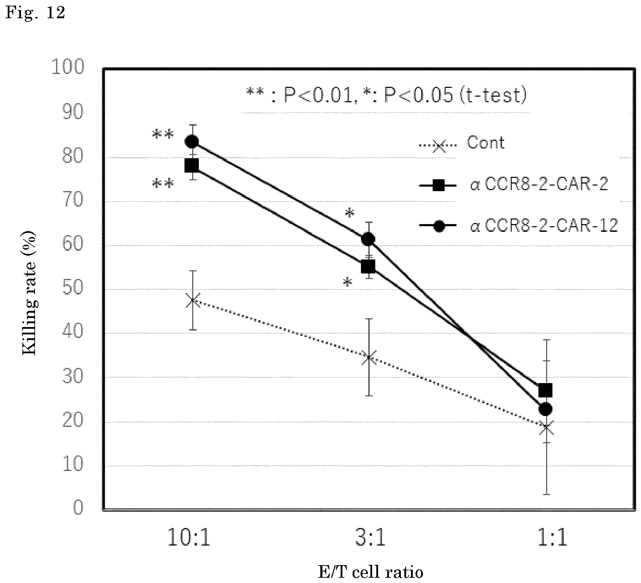

As a result, it was found that both of the peripheral blood-derived T cells expressing αCCR8-2-CAR-2 or αCCR8-2-CAR-12 had significantly similar cytotoxic activity as compared to Amp mRNA-introduced peripheral blood-derived T cells as a negative control (Figure 12).

Next, in the same manner, the E/T cell ratio was set to 5 : 1, and cytotoxic activity of the peripheral blood-derived T cells expressing αCCR8-2-CAR-2 or αCCR8-2-CAR-12 against TALL-1 cells, which are a human ALL cell line, and CCRF-HSB2 cells was evaluated by a calcein method.

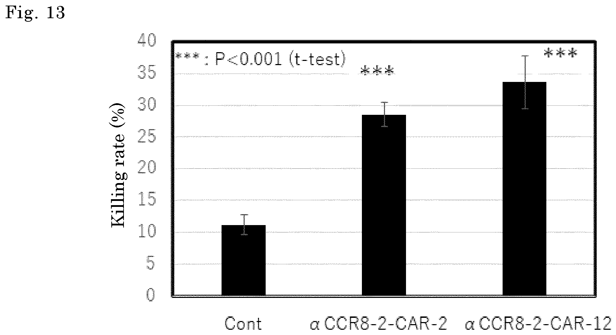

As a result, against the TALL-1 cells, the peripheral blood-derived T cells expressing αCCR8-2-CAR-2 or αCCR8-2-CAR-12 showed a significant increase in cytotoxic activity as compared to the Amp-introduced peripheral blood-derived T cells as a negative control (Figure 13). Similarly, for the CCRF-HSB2 cells, an increasing tendency of cytotoxic activity was observed.

Example 13: Evaluation of inhibitory activity of anti-CCR8 antibody against cytotoxic activity of anti-CCR8-CAR-expressing KHYG-1 cells

(1) Production of luciferase gene-expressing ATN-1 cells (Luc/ATN-1 cells)

A Sall/BglII fragment of a pGL3 vector (Clontech Laboratories, Inc.) was inserted into a Sall/BglII site of a pMEI-5 Neo vector to generate a vector expressing the firefly luciferase gene (pMEI5-FLuc). This expression vector was transduced into ATN-1 cells by electroporation using NEPA21. From 3 days after transduction, drug selection was performed with G418. Luciferase activity of drug-resistant cells was evaluated using Glo kit (Promega Corporation), and it was confirmed that the luciferase gene was expressed.

(2) Introduction of anti-CCR8-CAR gene into KHYG-1 cells by retrovirus

A gene fragment (SEQ ID NO: 62) of αCCR8-2-CAR-12 IRES-Puromycin was inserted into a NotI site of a pMEI-5 vector, which is a retroviral vector, to generate a vector capable of expressing αCCR8-2-CAR-12-IRES-Puromysin mRNA. The retrovirus was obtained by simultaneously transfecting 293GP2 cells with an αCCR8-2-CAR-12 retroviral vector and a pE-Ampho amphotropic env expression vector (Takara Bio Inc.), which is a retroviral envelope protein expression vector. A 293GP2 cell supernatant containing the obtained retrovirus was added to KHYG-1 cells together with polybrene (final concentration of 10 pg/ml) to obtain infected cells. From 3 days after infection, drug selection was performed with puromycin (0.5 pg/ml) as they were to obtain puromycin-resistant cells. An expression level of αCCR8-2-CAR-12 in these cells was analyzed by a flow cytometer after staining with a PPE-labeled anti-Flag antibody. As a result, the CAR-positive rate was 86%.

From the above, it was found that αCCR8-2-CAR-12 was expressed on a surface of the KHYG-1 cells.

Cytotoxic activity against Luc/ATN-1 cells was evaluated using αCCR8-2-CAR-12-expressing KHYG-1 cells by a calcein method as in Example 4.

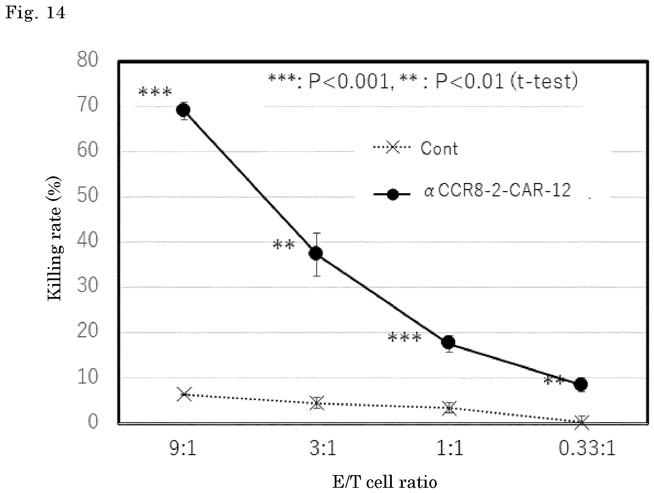

As a result, the αCCR8-2-CAR-12-expressing KHYG-1 cells showed significantly different potent cytotoxic activity against ATN-1 cells compared to KHYG-1 cells, which are negative control parent cells (Figure 14).

(3) Evaluation of inhibitory activity of anti-CCR8 antibody

It was examined whether or not cytotoxic activity by αCCR8-2-CAR-12-expressing KHYG-1 cells against ATN-1 cells was inhibited by an anti-CCR8 antibody having neutralizing activity. As the anti-CCR8 antibody, an αCCR8-1 antibody, which is an anti-CCR8 antibody shown in Table 1, and a BioLegend, Inc.'s anti-CCR8 antibody (L263G8) were used. Synagis was used as a negative control antibody. All the antibody concentrations were adjusted so that the final concentration was 33 nM. These antibodies were added to ATN-1 cells in advance, and 10 minutes later, effector cells, KHYG-1 cells or αCCR8-2-CAR-12-expressing KHYG-1 cells were added to evaluate cytotoxic activity. A calcein evaluation method was performed in the same manner as in Example 4. The results are displayed with a killing rate of an antibody non-added control as 100%.

As a result, cytotoxic activity of the ATN-1 cells by the KHYG-1 cells was hardly suppressed by any antibody. On the other hand, cytotoxic activity by the αCCRS-2-CAR-12-expressing KHYG-1 cells against the ATN-1 cells was significantly suppressed by 50% or more in both cases when the αCCR8-1 antibody and the L263G8 antibody were added as compared to the group to which no antibody was added or a negative control Synagis antibody was added.

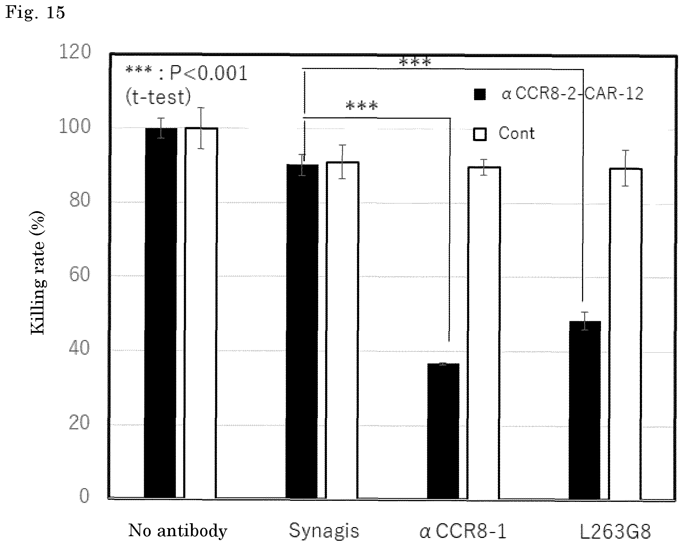

From the above results, it was found that ATN-1 cytotoxic activity by αCCR8-2-CAR-12-expressing KHYG-1 cells is mediated by a human CCR8 molecule expressed on an ATN-1 cell surface (Figure 15).

Example 14: Anti-tumor effect of anti-CCR8-CAR-expressing KHYG-1 cells in mice inoculated with human CCR8-expressing tumor cells

The back of SKID/Beige mice (female, 10 weeks old), which are immunodeficient mice, was subcutaneously inoculated with $5 \times 10^6$ human CCR8-expressing Rat-1 cells. On day 8 after inoculation, a tumor volume was measured and the mice were divided into 2 groups so as to have the same average value, and then $3 \times 10^6$ KHYG-1 cells as parent cells or the αCCR8-2-CAR-12-expressing KHYG-1 cells obtained in Example 13 per mouse were intratumorally administered (N = 3) at 30 μl on days 8, 13, and 16 after inoculation. The tumor volume and body weight were measured on days 8, 13, 16, 17, and 20 of inoculation of target cells. The tumor volume (mm$^3$) was measured in long diameter (mm) x short diameter (mm) x short diameter (mm)/2.

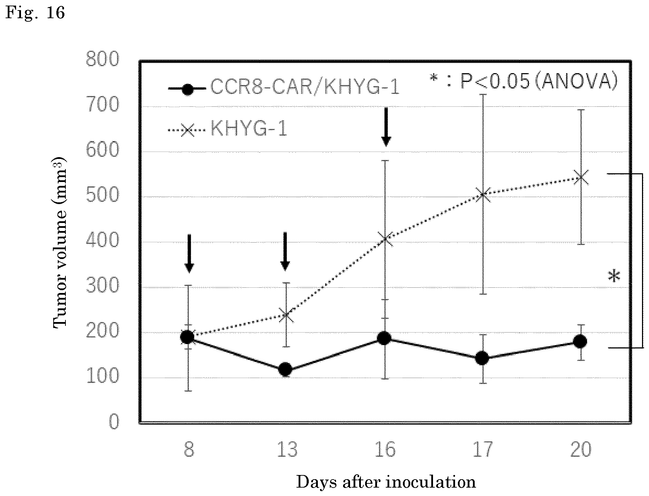

As a result, a rate of increase in tumor volume was significantly suppressed in the administration group of αCCR8-2-CAR-12-expressing KHYG-1 cells compared to the administration group of KHYG-1 cells, which are control parent cells (Figure 16). There was no difference in body weight change between both administration groups.

Example 15: Cytotoxic activity evaluation using iPS cell-derived NK cells expressing anti-CCR8-CAR

(1) Production of human iPS cell-derived NK cells

Using human iPS cells (TkDN4-M, Stem Cell Bank, The University of Tokyo), iPS cell-derived NK cells were produced by optimizing a culture method with reference to a known method (Cancer Science, 2020, Vol. 111, p1478-1490). For the cultured differentiated cells, NK cell-specific surface antigens were confirmed by FACS analysis. In the cell group 2 weeks after induction of differentiation, expression of CD34 and CD45, which are hematopoietic progenitor markers, was confirmed, whereas in the cell group at the 5th week of induction of differentiation, expression of CD34 was decreased, while an expression level of CD45 also found in mature hematopoietic cells was increased. In addition, in the cell group at the 5th week of induction of differentiation, an expression rate of CD56 as an NK marker was 50% or more, and expression of NKG2D, KIR2D, and the like, which are important for cytotoxic activity, was similarly confirmed.

(2) Production of anti-CCR8-CAR-carrying lentiviral vector

DNA encoding the synthesized $\alpha$CCR8-2-CAR-2 was cloned into a multicloning site of a lentiviral vector pCDH - EF1- MCS (System Biosciences, LLC.) to obtain an $\alpha$CCR8-2-CAR-2 lentiviral vector.

Lenti-X 293T cells (Takara Bio Inc.) cultured in a DMEM medium (Sigma-Aldrich Co. LLC) supplemented with 10% FCS and Penicillin-Streptomycin (ThermoFisher Scientific, Inc.) at 37°C under 5% $CO_2$ were seeded on Poly-L-Lysine (Sigma-Aldrich Co. LLC)-treated dishes. Using ppPACKH1 Lentivector Packaging Kit (System Biosciences, LLC.), the vector was packaged according to the method described in the kit to obtain an $\alpha$CCR8-2-CAR-2-carrying lentiviral vector.

As a negative control, a viral vector was synthesized using only the lentiviral vector pCDH-EF1-MCS and the ppPACKH1 Lentivector Packaging Kit (System Biosciences, LLC.) according to the method described in the kit to obtain a negative control viral vector.

(3) Lentiviral vector introduction into human iPS cell-derived NK cells

Synperonic F108 (Sigma-Aldrich Co. LLC), which is a lentivirus introduction adjuvant, was added to a final concentration of 1 mg/ml, and the $\alpha$CCR8-2-CAR-2-carrying lentiviral vector and the negative control viral vector were further added to human iPS cell-derived NK cells at 20,000 vps/cell, followed by culturing at 37°C under 5% $CO_2$ for approximately 24 hours.

The gene-introduced cells were used for evaluation of cytotoxic activity against Rat-1 cells expressing human CCR8 as target cells within approximately 24 hours after introduction.

(4) Analysis of anti-CCR8-CAR expression of human iPS cell-derived NK cells

Approximately 24 hours after gene introduction of human iPS cell-derived NK cells, an expression rate of an anti-CCR8-CAR on a cell surface was quantified using an anti-FLAG antibody (PE-anti-DYKDDDDK Tag Clone: L5, BioLegend, Inc.). The cells were stained with Zombie NIR Fiable Viability Kit (BioLegend, Inc.), an anti-FLAG antibody, and an anti-CD56 antibody (Brilliant Violet 421-anti-human CD56 clone: HCD56, BioLegend, Inc.) for 1 hour at room temperature, washed twice with a buffer for cytotoxic activity evaluation (DMEM/4% FCS), and then a cell surface expression rate of the anti-CCR8-CAR was evaluated by a flow cytometer. As a result, about 20% of $\alpha$CCR8-2-CAR-2 was expressed on a surface of human iPS cell-derived NK cells.

(5) Labeling human CCR8-expressing cells with calcein

Human CCR8-expressing cells to be labeled were suspended in a DMEM/10% FCS medium so as to be 1.8 $\times$ 10$^6$ cells/3 ml or less, and calcein (Calcein-AM, 349-07201, DOJINDO LABORATORIES) dissolved in DMSO was added so as to have a final concentration of 10 pg/ml. The cell culture solution was suspended, and then cultured at 37°C under 5% $CO_2$ for 30 minutes. Thereafter, the cells were washed twice with a buffer for cytotoxic activity evaluation (DMEM/4% FCS) and used for cytotoxic activity evaluation.

(6) Cytotoxic activity of human CCR8-expressing cells by anti-CCR8-CAR-introduced iPS cell-derived NK cells

24 hours after gene introduction into iPS cell-derived NK cells, the gene-introduced iPS cell-derived NK cells were mixed with the calcein-labeled human CCR8-expressing Rat-1 cells produced in the above (5) as target cells in a buffer for cytotoxic activity evaluation so that a ratio of the gene-introduced iPS cell-derived NK cells to the target cells (E/T cell ratio) was 10 : 1 at maximum, and the mixture was serially diluted so as to have an E/T cell ratio of 3 times the common ratio.

Cytotoxic activity was evaluated using U-bottomed 96 wells. After mixing the gene-introduced iPS cell-derived NK cells and the target cells, they were centrifuged using a plate centrifuge at 2000 rpm for 5 minutes to allow the cells to settle. The cells were mixed, and then cultured at 37°C under 5% $CO_2$ for 30 minutes. Thereafter, the mixture was centrifuged at 2000 rpm for 5 minutes using a plate centrifuge, and 70 $\mu$l of the cell supernatant was slowly separated from the plate. For the separated supernatant, an amount of fluorescence contained in the supernatant was quantified using a fluorescence measuring instrument within 30 minutes.

A spontaneous release amount (BG) of calcein from each target cell was determined by measuring the supernatant of the well of each target cell only in the same manner. A total calcein release amount (TOTAL) was determined by adding 1% Triton X-100 to the well of each target cell only so that Triton X-100 was 0.1% and measuring the same amount of supernatant.

As a method for calculating cytotoxic activity by using an amount of calcein released from a killed target cell as an index, calculation was performed by the following formula.

$$\{(\text{Fluorescence value of each well} - \text{BG fluorescence value})/((\text{TOTAL fluorescence value} - \text{BG fluorescence value})\} \times 100\ (\%)$$

Spontaneous release was subtracted, and assuming that an amount of fluorescence in the supernatant when all cells were killed (in this case, an amount of fluorescence released to the supernatant by complete cell lysis by Triton) was 100%, a killing rate (%) was calculated.

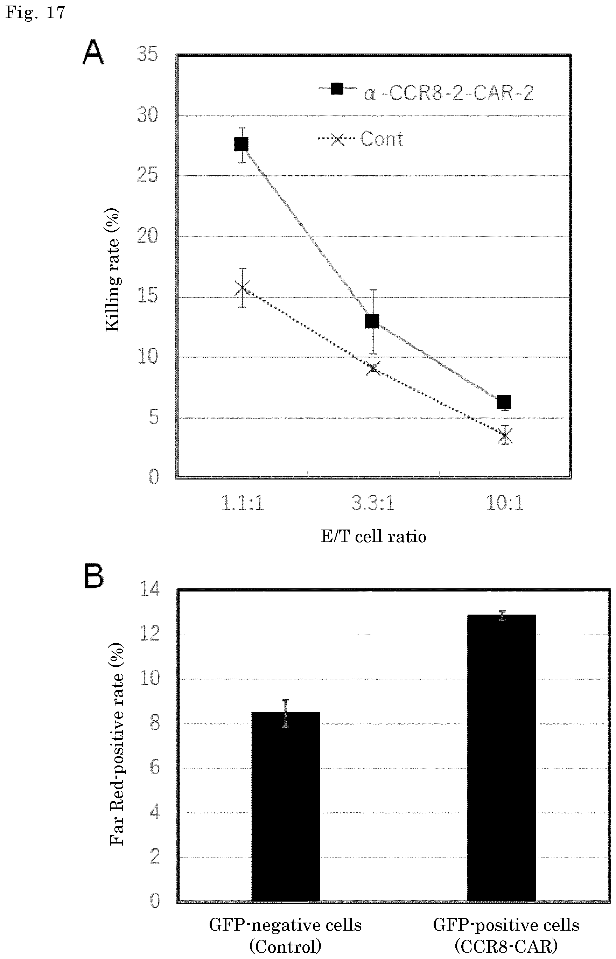

The results are shown in Figure 17A. As compared to the case where the negative control was genetically introduced, in the iPS cell-derived NK cells into which $\alpha$CCR8-2-CAR-2 was genetically introduced, human CCR8-expressing cell-dependent and anti-CCR8-CAR-expressing cell-specific cytotoxic activity was observed.

Example 16: Cytotoxic activity evaluation using iPS cell-derived macrophages expressing anti-CCR8-CAR

(1) Culturing into human iPS cell-derived macrophages

Human iPS cells (TkDN4-M, Stem Cell Bank, The University of Tokyo) were optimized with reference to a known method (Mol. Ther. Nucleic Acids, 2018, Vol. 12, p793 -804) to produce iPS cell-derived macrophages. For the cultured differentiated cells, macrophage-specific surface antigens were confirmed by FACS analysis. In the cell group 36 days after induction of differentiation, expression of CD34, which is a hematopoietic progenitor cell marker, was decreased, while an expression level of CD45 also found in mature hematopoietic cells was increased. In addition, expression of CD11b and CD14, which are surface antigens peculiar to macrophages, was confirmed.

(2) Introduction of mRNA of anti-CCR8-CAR into human iPS cell-derived macrophages

After washing the iPS cell-derived macrophages with PBS(-), 0.05% Trypsin-EDTA was recovered by treatment at 37°C under 5% $CO_2$ for 20 minutes. The recovered cells were washed with a medium, and then suspended in a solution of P3 Primary Cell 4D-Nucleofector X Kit (LONZA KK.) so as to be $5 \times 10^5$ cells/100 $\mu$l. 0.25 $\mu$l (0.5 pg) of each of $\alpha$CCR8-2-CAR-2 mRNA and negative control mRNA was added to Single Nucleocuvette (100 $\mu$l) containing 100 $\mu$l of the cell solution together with 0.1 $\mu$l (0.1 $\mu$g) of pmaxGFP Vector (LONZA KK.), and a normal human macrophage gene introduction protocol of a 4D-Nucleofector device was selected to perform gene introduction. The cells after mRNA introduction were cultured at 37°C under 5% $CO_2$ for 16 to 18 hours. The transformed cells were used for evaluation of cytotoxic activity against human CCR8-expressing cells as target cells within 20 hours after mRNA introduction.

(3) Labeling of human CCR8-expressing cells

Human CCR8-expressing cells to be labeled were collected by CTS TrypLE Select Enzyme (Gibco), washed once with a DMEM medium without serum addition, and suspended in a DMEM medium without serum addition so as to be $1 \times 10^6$ cells/ml or less. CellTrace Far Red staining solution was added so as to dilute 1000 times, and the mixture was gently stirred and allowed to stand in a thermostatic chamber at 37°C for 20 minutes. An equal amount of a DMEM/10% FCS medium was added, and the mixture was centrifuged, washed once with an iPS cell-derived macrophage culture medium, then suspended in an iPS cell-derived macrophage culture medium so as to be $1.5 \times 10^6$ cells/ml, and used for cytotoxic activity evaluation.

(4) Cytotoxic activity evaluation of human CCR8-expressing cells by anti-CCR8-CAR-introduced iPS cell-derived macrophages

To verify cytotoxic activity of the iPS cell-derived macrophages against human CCR8-expressing Rat-1 cells, cell phagocytic activity was evaluated.

20 hours after the iPS cell-derived macrophages were transformed, the transformed iPS cell-derived macrophages were mixed with the human CCR8-expressing Rat-1 cells stained with the CellTrace Far Red staining solution produced in the above (3) as target cells in 200 $\mu$l of an iPS cell-derived macrophage culture medium so that a ratio of functional cells/target cells (E/T cell ratio), which is a ratio of the transformed iPS cell-derived macrophages to the human CCR8-expressing Rat-1 cells, was 3 : 1 ($1.5 \times 10^5$ cells : $5.0 \times 10^4$ cells). The mixed solution was added to a 96-well round-bottom plate, and centrifuged at 100 g for 2 minutes using a plate centrifuge to allow the cells to settle, and the cells were cultured at 37°C under 5% $CO_2$ for 2 hours. Thereafter, the cells were centrifuged at 3000 rpm for 30 seconds and then the supernatant was removed, and the cells were suspended in 100 μl of an SFEB medium/1% BSA containing 0.05 pg/ml DAPI. Cell phagocytic activity against CCR8-expressing cells was detected by a flow cytometer.

As a method for calculating cell phagocytic activity, calculation was performed by the following formula.

$$\{(\text{GFP- and Far Red-co-positive cells})/(\text{GFP-positive cells})\} \times 100\ (\%)$$

As a result, as compared to the cells into which the negative control was genetically introduced, in the cells into which αCCR8-2-CAR-2 was genetically introduced, human CCR8-expressing cell-dependent and anti-CCR8-CAR-expressing cell-specific cytotoxic activity was observed.

Cytotoxic activity evaluation was also performed by another method in which (2) and subsequent methods were replaced with the following methods.

(2') Introduction of plasmid of anti-CCR8-CAR into human iPS cell-derived macrophages

After washing the iPS cell-derived macrophages with PBS(-), 0.05% Trypsin-EDTA was recovered by treatment at 37°C under 5% $CO_2$ for 20 minutes. The recovered cells were washed with a medium, and then suspended in a solution of P3 Primary Cell 4D-Nucleofector X Kit (LONZA KK.) so as to be $5 \times 10^5$ cells/100 μl. 0.2 μl (0.5 ug) of an αCCR8-2-CAR-2 vector was added to Single Nucleocuvette (100 μl) containing 100 μl of the cell solution together with 0.1 μl (0.1 μg) of pmaxGFP Vector (LONZA KK.), and a normal human macrophage gene introduction protocol of a 4D-Nucleofector device was selected to perform gene introduction. The cells after mRNA introduction were cultured at 37°C under 5% $CO_2$ for 16 to 18 hours. The transformed cells were used for evaluation of cytotoxic activity against human CCR8-expressing cells as target cells within 20 hours after plasmid introduction.

(3') Labeling of human CCR8-expressing cells

Human CCR8-expressing cells to be labeled were collected by CTS TrypLE Select Enzyme (Gibco), washed once with a DMEM medium without serum addition, and suspended in a DMEM medium without serum addition so as to be $1 \times 10^6$ cells/ml or less. CellTrace Far Red staining solution was added so as to dilute 1000 times, and the mixture was gently stirred and allowed to stand in a thermostatic chamber at 37°C for 20 minutes. An equal amount of a DMEM/10% FCS medium was added, and the mixture was centrifuged, washed once with an iPS cell-derived macrophage culture medium, then suspended in an iPS cell-derived macrophage culture medium so as to be $1.5 \times 10^6$ cells/ml, and used for cytotoxic activity evaluation.

(4') Cytotoxic activity evaluation of human CCR8-expressing cells by anti-CCR8-CAR-introduced iPS cell-derived macrophages

To verify cytotoxic activity of the iPS cell-derived macrophages against human CCR8-expressing Rat-1 cells, cell phagocytic activity was evaluated.

20 hours after the iPS cell-derived macrophages were transformed, the transformed iPS cell-derived macrophages were mixed with the human CCR8-expressing Rat-1 cells stained with the CellTrace Far Red staining solution produced in the above (3') as target cells in 200 μl of an iPS cell-derived macrophage culture medium so that a ratio of functional cells/target cells (E/T cell ratio), which is a ratio of the transformed iPS cell-derived macrophages to the human CCR8-expressing Rat-1 cells, was 3 : 1 ($1.5 \times 10^5$ cells : $5.0 \times 10^4$ cells). The mixed solution was added to a 96-well round-bottom plate, and centrifuged at 100 g for 2 minutes using a plate centrifuge to allow the cells to settle, and the cells were cultured at 37°C under 5% $CO_2$ for 2 hours. Thereafter, the cells were centrifuged at 3000 rpm for 30 seconds and then the supernatant was removed, and the cells were suspended in 100 μl of an SFEB medium/1% BSA containing 0.05 pg/ml DAPI. Cell phagocytic activity against CCR8-expressing cells was detected by a flow cytometer.

As a method for calculating cell phagocytic activity, calculation was performed as follows using GFP-positive cells in which gene introduction occurred and GFP-negative cells in which gene introduction did not occur for αCCR8-2-CAR-2.

- Cell phagocytic activity of GFP-positive cells:

$$\{(\text{GFP- and Far Red-co-positive cells})/(\text{GFP-positive cells})\} \times 100\ (\%)$$

- Cell phagocytic activity of GFP-negative cells:

$$\{(\text{GFP-negative and Far Red-positive cells})/(\text{GFP-negative cells})\} \times 100 \ (\%)$$

The results are shown in Figure 17B. As compared to the GFP-negative cells as a negative control, in the GFP-positive cells into which αCCR8-2-CAR-2 was genetically introduced, a significant increase in cytotoxic activity was observed (Student t-test, P < 0.005).

Example 17: Evaluation of cytotoxic activity of anti-CCR8-CAR-expressing NK cells against human ovarian cancer-derived tumor-infiltrating Treg cells

(1) Preparation of human ovarian cancer-derived tumor-infiltrating Treg cells

Tumor tissues derived from human ovarian cancer patients were dispersed using Tumor Dissociation Kit, human (Miltenyi Biotec) to prepare a cell population containing Treg cells, CD8-positive T cells, and CD4-positive T cells other than Treg cells. For the experiment, cells immediately after preparation or after cryopreservation were used.

(2) Measurement of CCR8-positive rate of human ovarian cancer-derived tumor-infiltrating Treg cells

For the cell population obtained in the above (1), a CCR8-positive rate in tumor-infiltrating Treg cells was measured using flow cytometry. The cells were suspended in an HBSS/2% FCS/10 mM HEPES buffer to which Human TruStain FcX (BioLegend, Inc.) and Zombie NIR Fixable Viability Kit (BioLegend, Inc.) were added, and the suspension was allowed to stand at 4°C for 30 minutes. After washing, various antibodies were added, and the mixture was allowed to stand at 4°C for 30 minutes (Bv785-labeled anti-human CD45 antibody, Alexa Flour 700-labeled anti-human CD3 antibody, Bv711-labeled anti-human CD4 antibody, PE-labeled anti-human CCR8 antibody, Bv605-labeled anti-human CD56 antibody, Bv510-labeled anti-human CD8 antibody (all manufactured by BioLegend, Inc.)). After washing, fixation and membrane permeation treatment were performed using eBioscience Foxp3/Transcription Factor Staining Buffer Set (Thermo Fisher Scientific Inc.), APC-labeled anti-human Foxp3 (Thermo Fisher Scientific Inc.) was added, and the mixture was allowed to stand at 4°C for 30 minutes. After washing, a CCR8-positive rate in tumor-infiltrating Treg cells (CD45-, CD3-, CD4-, and Foxp3-positive and Zombie NIR-, CD8-, and CD56-negative fractions) was measured using a flow cytometer.

(3) Evaluation of cytotoxic activity of human tumor-infiltrating Treg cells by anti-CCR8-CAR-expressing KHYG-1 cells

The human ovarian cancer-derived tumor-infiltrating Treg cells obtained in the above (1) were used as target cells, and KHYG-1 cells transformed with αCCR8-2-CAR-2 mRNA or negative control mRNA were used as effector cells. In the same manner as in Example 5, an αCCR8-2-CAR-2 expression rate on a KHYG-1 cell surface at 20 hours after transformation was calculated to be 22%.

Cytotoxic activity was evaluated from 20 hours to 44 hours after transformation. The target cells and the effector cells were seeded on 96-well round-bottom plates at $1 \times 10^5$ cells/well, and RPMI 1640/10% FCS/1% PS was used as a culture solution.

After 24 hours from the start of the evaluation, the cells were collected and suspended in an HBSS/2% FCS/10 mM HEPES buffer to which Human TruStain FcX (BioLegend, Inc.) and Zombie NIR™ Fixable Viability Kit (BioLegend, Inc.) were added, and the suspension was allowed to stand at 4°C for 30 minutes. After washing, various antibodies were added, and the mixture was allowed to stand at 4°C for 30 minutes (Bv785-labeled anti-human CD45 antibody, Alexa Flour 700-labeled anti-human CD3 antibody, Bv605-labeled anti-human CD56 antibody, Bv711-labeled anti-human CD4, Bv510-labeled anti-human CD8 antibody (all manufactured by BioLegend, Inc.)). After washing, fixation and membrane permeation treatment were performed using eBioscience™ Foxp3/Transcription Factor Staining Buffer Set (Thermo Fisher Scientific Inc.), APC-labeled anti-human Foxp3 (Thermo Fisher Scientific Inc.) was added, and the mixture was allowed to stand at 4°C for 30 minutes.

After washing, a proportion of tumor-infiltrating Treg cells (CD45-, CD3-, CD4-, and Foxp3-positive and Zombie NIR-, CD8-, and CD56-negative fractions) in surviving CD45-positive cells was quantified using a flow cytometer.

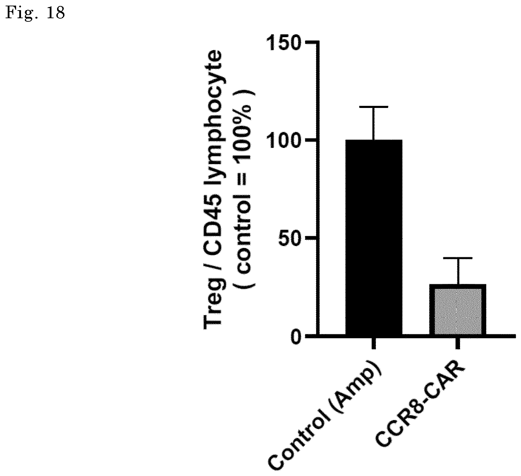

As a result, as compared to the negative control mRNA-introduced cells, a significant decrease in tumor-infiltrating Treg cells was observed in the αCCR8-2-CAR-2-expressing KHYG-1 cells (Figure 18, Student t-test, P < 0.005). In addition, the decrease width almost matched the CCR8-positive rate of the tumor-infiltrating Treg cells.

From the above, it was confirmed that uCCR8-2-CAR-2-expressing KHYG-1 cells have potent cytotoxic activity against tumor-infiltrating Treg cells expressing human CCR8.

(4) Evaluation of cytotoxic activity of human tumor-infiltrating Treg cells by peripheral blood-derived NK cells expressing anti-CCR8-CAR

The human ovarian cancer-derived tumor-infiltrating Treg cells obtained in the above (1) were used as target cells, and the peripheral blood-derived NK cells transformed with αCCR8-2-CAR-2 mRNA or negative control mRNA obtained in Example 11 were used as effector cells.

20 hours after transformation, the cells were collected and suspended in an HBSS/2% FCS/10 mM HEPES buffer to which Human TruStain FcX (BioLegend, Inc.) and Zombie NIR Fixable Viability Kit (BioLegend, Inc.) were added, and the suspension was allowed to stand at 4°C for 30 minutes. After washing, various antibodies were added, and the mixture was allowed to stand at 4°C for 30 minutes (Bv785-labeled anti-human CD45 antibody, Alexa Flour 700-labeled anti-human CD3 antibody, Bv605-labeled anti-human CD56 antibody, PE-labeled anti-FLAG antibody (all manufactured by BioLegend, Inc.)). After washing, an αCCR8-2-CAR-2 expression rate was calculated using a flow cytometer and found to be 5%. Cytotoxic activity was evaluated in the same manner as in the above (3).

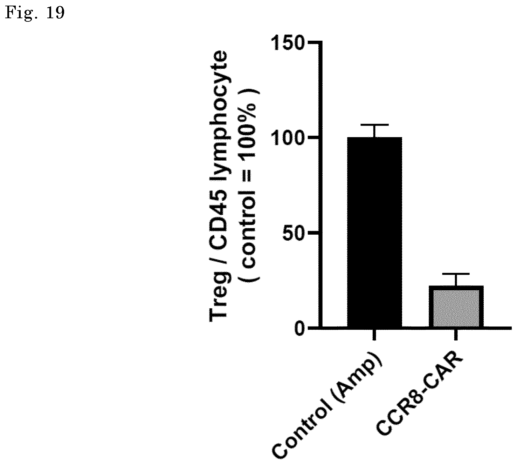

As a result, as compared to the negative control mRNA-introduced peripheral blood-derived NK cells, a significant decrease in tumor-infiltrating Treg cells was observed in the αCCR8-2-CAR-2-mRNA-introduced peripheral blood-derived NK cells. (Figure 19, Student t-test, P < 0.005). In addition, since the decrease width almost matched the CCR8-positive rate of the tumor-infiltrating Treg cells, it was confirmed that peripheral blood-derived NK cells expressing αCCR8-2-CAR-2 have potent cytotoxic activity against tumor-infiltrating Treg cells expressing CCR8.

Example 18: Evaluation of cytotoxic activity of anti-CCR8-CAR-expressing NK cells against human colorectal cancer-derived tumor-infiltrating Treg cells

Tumor-infiltrating Treg cells obtained from tumor tissues derived from human colorectal cancer patients in the same manner as in Example 17(1) were used as target cells, and the peripheral blood-derived NK cells transformed with αCCR8-2-CAR-2 mRNA or negative control mRNA obtained in Example 11 were used as effector cells. After seeding the cells in the same manner as in Example 17(3), on day 5, an abundance ratio of the tumor-infiltrating Treg cells and IFNy production of CD8-positive T cells and CD4-positive T cells other than the tumor infiltrating Treg cells were quantified using a flow cytometer. After the cells were collected, the cells were stained with an IFNy detection reagent (IFN-γ Secretion Assay-Detection Kits, human, Miltenyi Biotech) and various labeled antibodies of Example 17(3).

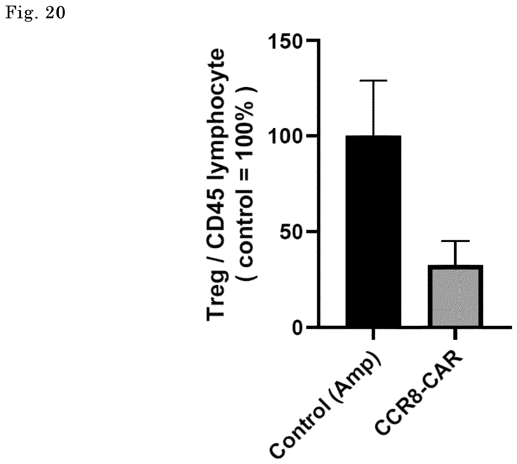

As a result of measurement using a flow cytometer, as compared to the negative control mRNA-introduced peripheral blood-derived NK cells, a significant decrease in tumor-infiltrating Treg cells was observed in the αCCR8-2-CAR-2-mRNA-introduced peripheral blood-derived NK cells (Figure 20, Student t-test, P < 0.005). In addition, an IFNy-positive rate of CD8-positive T cells and CD4-positive T cells other than the tumor-infiltrating Treg cells tended to increase.

From the above, it was confirmed that peripheral blood-derived NK cells expressing αCCR8-2-CAR-2 have CCR8-expressing tumor-infiltrating Treg cell-depleting activity and a T cell activating effect.

Example 19: Evaluation of anti-tumor activity of anti-CCR8-CAR-expressing KHYG-1 cells in mice inoculated with human CCR8-expressing tumor cells (ATL cell line-derived ATN-1 cells)

NOG mice (29 weeks old, female) knocked-in with human IL-15 were intraperitoneally inoculated with $1 \times 10^7$ (100 μl) ATN-1 cells into which a luciferase gene was introduced. The ATN-1 cells are a cell line that naturally expresses human CCR8.

After 2 and 5 days from tumor inoculation, in the αCCR8-2-CAR-12-expressing KHYG-1 cell administration group, $2 \times 10^6$ (100 μl) αCCR8-2-CAR-12-expressing KHYG-1 cells obtained in Example 13 were intraperitoneally administered, and in the control group, $2 \times 10^6$ (100 μl) KHYG-1 cells were intraperitoneally administered (N = 7). After 2, 7, 12, and 14 days from tumor inoculation, 100 μl of a D-Luciferin (Summit Pharmaceuticals International Corporation) PBS diluted solution (15 mg/ml) was intravenously administered to the mice, and luminescence intensity of tumor was measured using IVIS Spectrum (PerkinElmer, Inc.). The luminescence intensity was calculated by subtracting a measured value of back ground from a measured real value, and the measurement was performed under conditions of exposure time: 5 seconds, binning: low, and F/Stop: 1. The luminescence intensity is a numerical value obtained by quantifying the total luminescence intensity within a range of ROI, and an ROI area at each measurement was set to be the same. As software for analyzing a luminescence image, living image ∘R 3.2 was used. A Mann-Whitney U method was used for a significant difference test (significance level: P < 0.05).

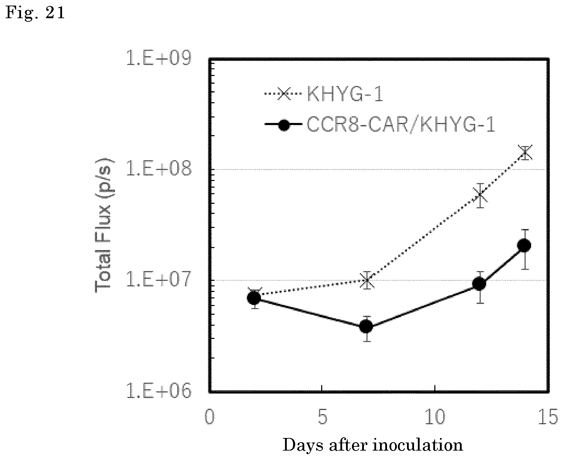

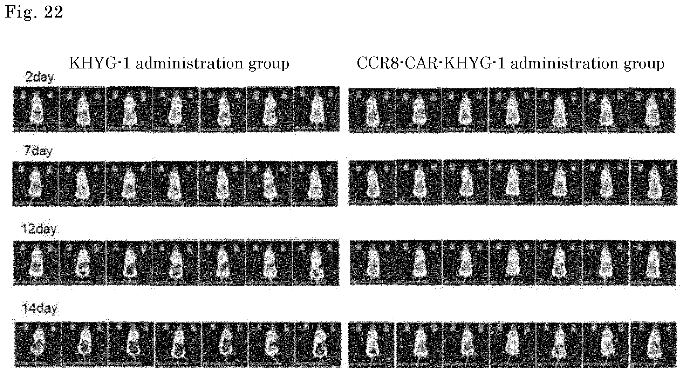

As a result, after 7, 12, and 14 days from tumor inoculation, in the αCCR8-2-CAR-12-expressing KHYG-1 cell administration group, a significant decrease in luminescence intensity of tumor was observed as compared to the control group (Figures 21 and 22).

From the above, it was revealed that αCCR8-2-CAR-12-expressing KHYG-1 cells exhibit anti-tumor activity in mice inoculated with human CCR8-expressing tumor cells.

Example 20: Evaluation of anti-tumor activity of anti-CCR8-CAR-expressing KHYG-1 cells in human CCR8 knock-in mice (hCCR8-KI (KI/KI) mice) inoculated with colorectal cancer-derived CT26 cells

The back of hCCR8-KI (KI/KI) mice (13 weeks old, female) was intracutaneously inoculated with a CT26 cell line. After 7, 9, and 10 days from tumor inoculation, in the αCCR8-2-CAR-12-expressing KHYG-1 cell administration group, $2 \times 10^6$ (100 μl) αCCR8-2-CAR-12-expressing KHYG-1 cells obtained in Example 13 were intratumorally administered, and in the control group, $2 \times 10^6$ (100 μl) KHYG-1 cells were intratumorally administered (N = 7). Tumor volumes were measured every 3 to 4 days from 7 days after tumor inoculation. The tumor volume ($mm^3$) was measured in long diameter (mm) x short diameter (mm) x short diameter (mm)/2, and a significant difference test based on a Mann-Whitney U method (significance level: P < 0.05) was conducted.

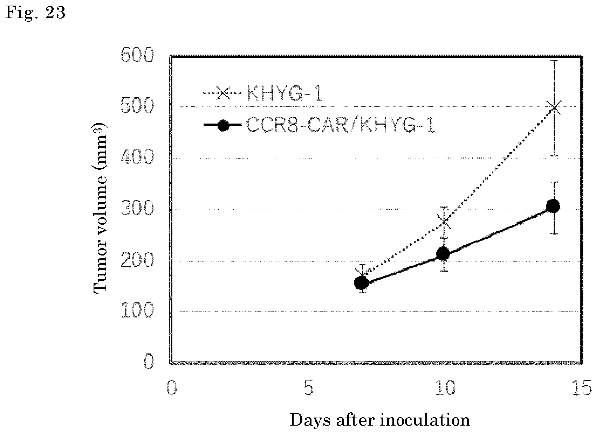

As a result, after 10 days from tumor inoculation, in the αCCR8-2-CAR-12-expressing KHYG-1 cell administration group, a significant decrease in tumor volume was observed as compared to the control group. Also after 14 days from tumor inoculation, a decreasing tendency was observed (Figure 23, P = 0.0973).

From the above, it was revealed that αCCR8-2-CAR-12-expressing KHYG-1 cells exhibit anti-tumor activity in hCCR8-KI (KI/KI) mice inoculated with colorectal cancer.

Example 21: Evaluation of cytotoxic activity of anti-CCR8-CAR-expressing KHYG-1 cells against tumor-infiltrating Treg cells in hCCR8-KI (KI/KI) mice inoculated with colorectal cancer-derived CT26 cells

The back of hCCR8-KI (KI/KI) mice (10 weeks old, female) was intracutaneously inoculated with a CT26 cell line. After 8 days from tumor inoculation, in the αCCR8-2-CAR-12-expressing KHYG-1 cell administration group, $2 \times 10^6$ (40 μl) αCCR8-2-CAR-12-expressing KHYG-1 cells obtained in Example 13 were intratumorally administered, and in the control group, $2 \times 10^6$ (40 μl) KHYG-1 cells were intratumorally administered (N = 5). After 1 day from cell administration, tumors were recovered from the mice. A tumor mass was finely cut with scissors, and a tumor-infiltrating cell suspension was prepared using Tumor Dissociation Kit, mouse (Miltenyi Biotec) according to the protocol attached to the kit. The cell suspension was stained using a Fixable Viability Dye eFluor 780 reagent (eBioscience, Inc.) at 4°C for 30 minutes. After washing once with 2% FCS/10 mM HEPES/HBSS, the cells were stained with a non-fluorescently labeled anti-human CCR8 antibody (or isotype control antibody) at 4°C for 30 minutes. After washing twice with 2% FCS/10 mM HEPES/HBSS, the cells were stained with an AF647-labeled anti-rat IgG antibody at 4°C for 30 minutes. The cells were stained with Bv510-labeled anti-mouse CD45, Bv605-labeled anti-mouse TCRβ, and Bv785-labeled anti-mouse CD4 antibodies at 4°C for 30 minutes. After washing with 2% FCS/HEPES/HBSS, the cells were fixed using Foxp3/Transcription Factor Staining Buffer Set (eBioscience, Inc.) according to the attached protocol, and intracellular FoxP3 was stained using a PE-labeled anti-mouse FoxP3 antibody. After washing with the buffer attached to the kit, T cells (CD45+ TCRβ+) were gated using a flow cytometer, and a CCR8-positive rate in tumor-infiltrating Treg cells (CD4+ Foxp3+) was analyzed. A Student t-test method was used for a significant difference test (significance level: P < 0.05). Staining antibodies used are shown below. As the anti-human CCR8 antibody, an antibody obtained based on the method described in Example 2 of WO 2020/138489 was used.

rat IgG2a isotype control antibody (Bio X Cell)
AF647 AffiniPure F(ab') Fragment Donkey Anti-Rat IgG(H+L) (Jackson ImmunoResearch Inc.)
PE anti-mouse/rat FoxP3 (clone FJK-16s, eBioscience, Inc.)
Bv510 anti-mouse CD45 (clone 30-F11, BioLegend, Inc.)
Bv605 anti-mouse TCRβ (clone PC61, BioLegend, Inc.)
Bv421 anti-mouse CD25 (clone PC61, BioLegend, Inc.)
Bv785 anti-mouse CD4 (clone RM4-5, BioLegend, Inc.)

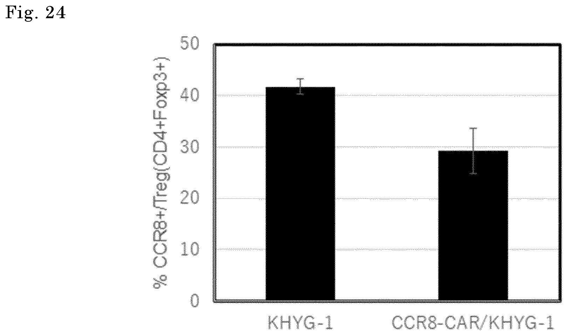

As a result, in the αCCR8-2-CAR-12-expressing KHYG-1 cell administration group, a significant decrease in CCR8-positive cells in tumor-infiltrating Treg cells was observed as compared to the control group (Figure 24).

From the above, it was revealed that αCCR8-2-CAR-12-expressing KHYG-1 cells have a tendency to remove tumor-infiltrating Treg cells expressing human CCR8 in mice in vivo.

[INDUSTRIAL APPLICABILITY]

The chimeric antigen receptor that recognizes CCR8 as an antigen of the present invention has cytotoxic activity against CCR8-expressing cells by being expressed in effector cells. In other words, the cell expressing the chimeric antigen receptor that recognizes CCR8 as an antigen of the present invention is very useful as a medicament for the treatment or prevention of CCR8-related diseases.

[SEQUENCE LISTING]

**Claims**

1. A chimeric antigen receptor comprising an extracellular region that binds to CCR8, a transmembrane region, and an intracellular region, wherein the intracellular region comprises an intracellular signal region of a signaling molecule expressed in a lymphocyte that functions as an effector cell.

2. The chimeric antigen receptor according to claim 1, wherein the extracellular region that binds to CCR8 comprises an anti-CCR8-scFv region.

3. The chimeric antigen receptor according to claim 2, wherein the anti-CCR8-scFv region comprises:

   1) a light chain variable region including

      CDR1 having the amino acid sequence of SEQ ID NO: 24,
      CDR2 having the amino acid sequence of SEQ ID NO: 25, and
      CDR3 having the amino acid sequence of SEQ ID NO: 26, and
      a heavy chain variable region including
      CDR1 having the amino acid sequence of SEQ ID NO: 27,
      CDR2 having the amino acid sequence of SEQ ID NO: 28, and
      CDR3 having the amino acid sequence of SEQ ID NO: 29;

   2) a light chain variable region including

      CDR1 having the amino acid sequence of SEQ ID NO: 30,
      CDR2 having the amino acid sequence of SEQ ID NO: 31, and
      CDR3 having the amino acid sequence of SEQ ID NO: 32, and
      a heavy chain variable region including
      CDR1 having the amino acid sequence of SEQ ID NO: 33,
      CDR2 having the amino acid sequence of SEQ ID NO: 28, and
      CDR3 having the amino acid sequence of SEQ ID NO: 34;

   3) a light chain variable region including

      CDR1 having the amino acid sequence of SEQ ID NO: 35,
      CDR2 having the amino acid sequence of SEQ ID NO: 36, and
      CDR3 having the amino acid sequence of SEQ ID NO: 37, and
      a heavy chain variable region including
      CDR1 having the amino acid sequence of SEQ ID NO: 38,
      CDR2 having the amino acid sequence of SEQ ID NO: 39, and
      CDR3 having the amino acid sequence of SEQ ID NO: 40;

   4) a light chain variable region including

      CDR1 having the amino acid sequence of SEQ ID NO: 30,
      CDR2 having the amino acid sequence of SEQ ID NO: 41, and
      CDR3 having the amino acid sequence of SEQ ID NO: 32, and
      a heavy chain variable region including
      CDR1 having the amino acid sequence of SEQ ID NO: 42,
      CDR2 having the amino acid sequence of SEQ ID NO: 28, and
      CDR3 having the amino acid sequence of SEQ ID NO: 43;

   5) a light chain variable region including

      CDR1 having the amino acid sequence of SEQ ID NO: 44,
      CDR2 having the amino acid sequence of SEQ ID NO: 45, and
      CDR3 having the amino acid sequence of SEQ ID NO: 46, and
      a heavy chain variable region including
      CDR1 having the amino acid sequence of SEQ ID NO: 47,

CDR2 having the amino acid sequence of SEQ ID NO: 28, and
CDR3 having the amino acid sequence of SEQ ID NO: 48; or

6) a light chain variable region including

CDR1 having the amino acid sequence of SEQ ID NO: 56,
CDR2 having the amino acid sequence of SEQ ID NO: 57, and
CDR3 having the amino acid sequence of SEQ ID NO: 58, and
a heavy chain variable region including
CDR1 having the amino acid sequence of SEQ ID NO: 59,
CDR2 having the amino acid sequence of SEQ ID NO: 60, and
CDR3 having the amino acid sequence of SEQ ID NO: 61.

4. The chimeric antigen receptor according to claim 3, wherein the anti-CCR8-scFv region comprises:

1) a light chain variable region including

CDR1 having the amino acid sequence of SEQ ID NO: 24,
CDR2 having the amino acid sequence of SEQ ID NO: 25, and
CDR3 having the amino acid sequence of SEQ ID NO: 26, and
a heavy chain variable region including
CDR1 having the amino acid sequence of SEQ ID NO: 27,
CDR2 having the amino acid sequence of SEQ ID NO: 28, and
CDR3 having the amino acid sequence of SEQ ID NO: 29;

2) a light chain variable region including

CDR1 having the amino acid sequence of SEQ ID NO: 30,
CDR2 having the amino acid sequence of SEQ ID NO: 31, and
CDR3 having the amino acid sequence of SEQ ID NO: 32, and
a heavy chain variable region including
CDR1 having the amino acid sequence of SEQ ID NO: 33,
CDR2 having the amino acid sequence of SEQ ID NO: 28, and
CDR3 having the amino acid sequence of SEQ ID NO: 34; or

4) a light chain variable region including

CDR1 having the amino acid sequence of SEQ ID NO: 30,
CDR2 having the amino acid sequence of SEQ ID NO: 41, and
CDR3 having the amino acid sequence of SEQ ID NO: 32, and
a heavy chain variable region including
CDR1 having the amino acid sequence of SEQ ID NO: 42,
CDR2 having the amino acid sequence of SEQ ID NO: 28, and
CDR3 having the amino acid sequence of SEQ ID NO: 43.

5. The chimeric antigen receptor according to claim 4, wherein the anti-CCR8-scFv region comprises:

a light chain variable region including
CDR1 having the amino acid sequence of SEQ ID NO: 24,
CDR2 having the amino acid sequence of SEQ ID NO: 25, and
CDR3 having the amino acid sequence of SEQ ID NO: 26, and
a heavy chain variable region including
CDR1 having the amino acid sequence of SEQ ID NO: 27,
CDR2 having the amino acid sequence of SEQ ID NO: 28, and
CDR3 having the amino acid sequence of SEQ ID NO: 29.

6. The chimeric antigen receptor according to claim 4, wherein the anti-CCR8-scFv region comprises:

a light chain variable region including
CDR1 having the amino acid sequence of SEQ ID NO: 30,
CDR2 having the amino acid sequence of SEQ ID NO: 31, and
CDR3 having the amino acid sequence of SEQ ID NO: 32, and
a heavy chain variable region including
CDR1 having the amino acid sequence of SEQ ID NO: 33,
CDR2 having the amino acid sequence of SEQ ID NO: 28, and
CDR3 having the amino acid sequence of SEQ ID NO: 34.

7. The chimeric antigen receptor according to claim 2 or 3, wherein the anti-CCR8-scFv region comprises:

1) a light chain variable region having the amino acid sequence of SEQ ID NO: 12, and
a heavy chain variable region having the amino acid sequence of SEQ ID NO: 13;
2) a light chain variable region having the amino acid sequence of SEQ ID NO: 14, and
a heavy chain variable region having the amino acid sequence of SEQ ID NO: 15; or
3) a light chain variable region having the amino acid sequence of SEQ ID NO: 16, and
a heavy chain variable region having the amino acid sequence of SEQ ID NO: 17.

8. The chimeric antigen receptor according to claim 7, wherein the anti-CCR8-scFv region comprises:

1) a light chain variable region having the amino acid sequence of SEQ ID NO: 12, and
a heavy chain variable region having the amino acid sequence of SEQ ID NO: 13; or
2) a light chain variable region having the amino acid sequence of SEQ ID NO: 14, and
a heavy chain variable region having the amino acid sequence of SEQ ID NO: 15.

9. The chimeric antigen receptor according to claim 8, wherein the anti-CCR8-scFv region comprises:

a light chain variable region having the amino acid sequence of SEQ ID NO: 12, and
a heavy chain variable region having the amino acid sequence of SEQ ID NO: 13.

10. The chimeric antigen receptor according to claim 8, wherein the anti-CCR8-scFv region comprises:

a light chain variable region having the amino acid sequence of SEQ ID NO: 14, and
a heavy chain variable region having the amino acid sequence of SEQ ID NO: 15.

11. The chimeric antigen receptor according to any one of claims 2 to 10, wherein the light chain variable region and the heavy chain variable region are linked via the linker sequence of SEQ ID NO: 2 in the anti-CCR8-scFv region.

12. The chimeric antigen receptor according to any one of claims 2 to 11, wherein the extracellular region that binds to CCR8 comprises a CD8A signal sequence region.

13. The chimeric antigen receptor according to claim 12, wherein the CD8A signal sequence region is present on an N-terminal side of the anti-CCR8-scFv region.

14. The chimeric antigen receptor according to claim 12 or 13, wherein the CD8A signal sequence region has the amino acid sequence of SEQ ID NO: 1.

15. The chimeric antigen receptor according to any one of claims 2 to 14, wherein the anti-CCR8-scFv region and the transmembrane region are linked via a spacer sequence.

16. The chimeric antigen receptor according to claim 15, wherein the spacer sequence comprises a Flag epitope-containing sequence region.

17. The chimeric antigen receptor according to claim 16, wherein the Flag epitope-containing sequence region has the amino acid sequence of SEQ ID NO: 3.

18. The chimeric antigen receptor according to any one of claims 15 to 17, wherein the spacer sequence comprises a CD8A hinge region.

19. The chimeric antigen receptor according to claim 18, wherein the CD8A hinge region has the amino acid sequence of SEQ ID NO: 4.

20. The chimeric antigen receptor according to any one of claims 1 to 19, wherein the transmembrane region comprises a CD8A transmembrane region.

21. The chimeric antigen receptor according to claim 20, wherein the CD8A transmembrane region has the amino acid sequence of SEQ ID NO: 5.

22. The chimeric antigen receptor according to any one of claims 1 to 19, wherein the transmembrane region comprises a CD28 transmembrane region and the intracellular region comprises a CD28 intracellular signal region.

23. The chimeric antigen receptor according to claim 22, wherein the CD28 transmembrane region has the amino acid sequence of SEQ ID NO: 6, and the CD28 intracellular signal region has the amino acid sequence of SEQ ID NO: 7.

24. The chimeric antigen receptor according to any one of claims 1 to 23, wherein the intracellular region comprises at least one of intracellular signal regions of 4-1BB, 2B4, CD3£, and DAP10.

25. The chimeric antigen receptor according to claim 24, wherein the intracellular region comprises at least two of intracellular signal regions of 4-1BB, 2B4, CD3£, and DAP 10.

26. The chimeric antigen receptor according to claim 25, wherein the intracellular region comprises intracellular signal regions of 4-1BB and CD3£.

27. The chimeric antigen receptor according to claim 25, wherein the intracellular region comprises intracellular signal regions of 2B4 and CD3£.

28. The chimeric antigen receptor according to any one of claims 24 to 27, wherein the intracellular region comprises a 4-1BB intracellular signal region, and the 4-1BB intracellular signal region has the amino acid sequence of SEQ ID NO: 8.

29. The chimeric antigen receptor according to any one of claims 24 to 27, wherein the intracellular region comprises a 2B4 intracellular signal region, and the 2B4 intracellular signal region has the amino acid sequence of SEQ ID NO: 9.

30. The chimeric antigen receptor according to any one of claims 24 to 27, wherein the intracellular region comprises a CD3£ intracellular signal region, and the CD3£ intracellular signal region has the amino acid sequence of SEQ ID NO: 10.

31. The chimeric antigen receptor according to claims 24 to 27, wherein the intracellular region comprises a DAP10 intracellular signal region, and the DAP10 intracellular signal region has the amino acid sequence of SEQ ID NO: 11.

32. An effector cell expressing the chimeric antigen receptor according to any one of claims 1 to 31.

33. The cell according to claim 32, wherein the effector cell is an NK cell.

34. The cell according to claim 32, wherein the effector cell is a T cell.

35. The cell according to claim 32, wherein the effector cell is a macrophage.

36. The cell according to any one of claims 32 to 35, wherein the effector cell is derived from peripheral blood.

37. The cell according to any one of claims 32 to 35, wherein the effector cell is differentiated from a stem cell.

38. The cell according to claim 36, wherein the stem cell is an iPS cell.

39. A pharmaceutical composition comprising the cell according to any one of claims 32 to 38.

40. The pharmaceutical composition according to claim 39, for use in treating cancer.

41. The pharmaceutical composition according to claim 40, having an effect of depleting tumor-infiltrating Treg cells expressing CCR8.

42. The pharmaceutical composition according to claim 40, having an effect of depleting tumor cells expressing CCR8.

43. A polynucleotide encoding an amino acid sequence of the chimeric antigen receptor according to any one of claims 1 to 31.

44. An expression vector comprising the polynucleotide according to claim 43.

Fig. 1

| | VL | VH |
|---|---|---|
| α CCR8-1<br>VL:<br>Seq. No. 12<br>VH:<br>Seq. No. 13 | DIVMTQSPDSLAVSLGERATIN**CRSSKSLLHSNRNTYLY**WYQQKPGQPPKLLIY**RMSQLAS**GVPDRFSGSGSGTDFTLTISSLQAEDVAVYYC**MQHLEYPLT**FGQGTKLEIK | EVQLVESGGALVKPGGSLRLSCAASGFTFS**TYALY**WVRQAPGKGLEWVG**RIRSKSNNYATYYADSVKD**RFTISRDDSKNTLYLQMNSLKTEDTAVYYCTR**ARFYYSDYGYAMDY**WGQGTLVTVSS |
| α CCR8-2<br>VL:<br>Seq. No. 14<br>VH:<br>Seq. No. 15 | EIVLTQSPGTLSLSPGERATLS**CRSSKSLLHSNGNTYLY**WYQQKPGQAPRLLIY**RVSNLAS**GIPDRFSGSGSGTDFTLTISRLEPEDFAVYYC**MQHLEYPFT**FGQGTKLEIK | EVQLVESGGALVKPGGSLRLSCAASGFTFS**TYAMY**WVRQAPGKGLEWVG**RIRSKSNNYATYYADSVKD**RFTISRDDSKNTLYLQMNSLKTEDTAVYYCTR**GGYGNYRYAMDY**WGQGTTVTVSS |
| α CCR8-3<br>VL:<br>Seq. No. 16<br>VH:<br>Seq. No. 17 | DVVLTQTPLSLPVSLGGQASIS**CRSSQSLVHSNGNTYLH**WYLQKPGQSPKLLIY**KVSNRFS**GVPDRFSGSGSGTDFTLKISRVEAEDLGVYFC**SQSTQVPLT**FGAGTKLELKR | QVQLKQSGPSLVQPSQSLSITCTVSGFSLT**AYGVH**WIRQSPGKGQEWLG**VIWRGGSTDYNAAFMSR**LTITKDNSKSQVFFKMNSLQADDTAIYYCAK**KGGSYAMDY**WGQGTSVTVSS |
| α CCR8-4<br>VL:<br>Seq. No. 18<br>VH:<br>Seq. No. 19 | DIVMTQAAPSVPVTPGESVSIS**CRSSKSLLHSNGNTYLY**WFLQRPGQSPQLLIY**RMSNLAS**GVPDRFSGSGSGTAFTLRISRVEAEDVGVYYC**MQHLEYPFT**FGSGTKLEIKRA | EVQLVETGGGLVQPKGSLKLSCAASGFTFNT**NAMN**WVRQAPGKGLEWVA**RIRSKSNNYATYYADSVKD**RFTISRDDSQSMLYLQMNNLKTEDTAMYYCVR**EIRPLGDFDV**WGAGTTVTVSS |
| α CCR8-5<br>VL:<br>Seq. No. 20<br>VH:<br>Seq. No. 21 | EIVMTQAVPSVPVTPGESVSIS**CRSSKSLLHSNGNTYLY**WFLQRPGQSPQLLIY**RMSNLAS**GVPDRFSGSGSGTAFTLRISRVEAEDVGVYYC**MQHLEYPFT**FGSGTKLEIKRAD | EVQLVETGGGLVQPKGSLKLSCAASGFTFNT**NAMN**WVRQTPGKGLEWVA**RIRSKSNNYATYYADSVKD**RFTISRDDSQSMLYLQMNNLKTEDTAVYYCVR**EIRPLGDFDV**WGAGTTVTVSS |
| α CCR8-6<br>VL:<br>Seq. No. 22<br>VH:<br>Seq. No. 23 | ETTVTQSPASLSMAIGEKVTIR**CITSTDIDDDMN**WYQQKPGEPPKLLIS**EGNTLRP**GVPSRFSSSGYGTDFVFTIENMLSEDVADYYC**LQSDNLPYT**FGGGTKLEIKRA | EVQLVESGGGLVQPKGSLKLSCAASGFTFN**AYAMN**WVRQAPGKGLEWIA**RIRSKSNNYATYYADSVKD**RFTISRDDSQSMLYLQMNNLKTEDTAMYYCVR**HKFGRNYYAMDY**WGQGTSVTVSS |
| α CCR8-7<br>VL:<br>Seq. No. 54<br>VH:<br>Seq. No. 55 | DIVLTQSPALAVSLGQ**RATISCRASQSVSISRYNIMH**WYQQKPGQQPKLLIY**RASNLAS**GIPARFSGSGSGTDFTLTINPVQADDIATYYC**QQSRESPPT**FGGGTKLELKR | EVQLVESDGGLVQPGRSLKLSCAASGFTFS**DYYMA**WVRQAPTKGLEWVA**TITYDVYSTYYRDSVKG**RFTISRDNAKSTLYLQMDSLRSEDTATYYCAR**HGGDGYNSGNWFAY**WGQGTLVTVSS |

Fig. 2

CD8A signal sequence region

G4S peptide linker

Anti-CCR8-scFV region

Extracellular region that binds to CCR8

Heavy chain variable region

Light chain variable region

Spacer sequence region

Transmembrane region

Intracellular region

Fig. 3

Fig. 4

Fig. 5

Fig. 6

Fig. 7

Fig. 8

Fig. 9

A

|  | Isotype antibody Control | Anti-CCR8 Control | Anti-CCR8 No2-CAR |

B

Killing rate (%)

···×·· Control

■ α CCR8-2-CAR

E/T cell ratio

Fig. 10

Fig. 11

Fig. 12

Fig. 13

Fig. 14

Fig. 15

Fig. 16

Days after inoculation

Fig. 17

Fig. 18

Fig. 19

Fig. 20

Fig. 21

Fig. 22

KHYG-1 administration group        CCR8-CAR-KHYG-1 administration group

Fig. 23

Fig. 24

## INTERNATIONAL SEARCH REPORT

| International application No. |
|---|
| **PCT/JP2022/016625** |

**A. CLASSIFICATION OF SUBJECT MATTER**

*C12N 15/62*(2006.01)i; *A61K 35/17*(2015.01)i; *A61P 35/00*(2006.01)i; *C07K 14/725*(2006.01)i; *C07K 16/28*(2006.01)i; *C07K 19/00*(2006.01)i; *C12N 5/10*(2006.01)i; *C12N 15/12*(2006.01)i; *C12N 15/13*(2006.01)i; *C12N 15/63*(2006.01)i
FI: C12N15/62 Z ZNA; C12N15/13; C12N15/12; C12N15/63 Z; C07K19/00; C07K14/725; C07K16/28; C12N5/10; A61K35/17 Z; A61P35/00

According to International Patent Classification (IPC) or to both national classification and IPC

**B. FIELDS SEARCHED**

Minimum documentation searched (classification system followed by classification symbols)

C12N15/00-15/90; C07K1/00-19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/138489 A1 (SHIONOGI & CO., LTD.) 02 July 2020 (2020-07-02)<br>claims, paragraphs [0001]-[0012], [0063]-[064], examples, fig. 2-7 | 1-44 |
| Y | CAMPBELL, J. R. et al. Fc-Optimized Anti-CCR8 Antibody Depletes Regulatory T cells in Human Tumor Models. Cancer Res. 2021, 81(11), pp. 2983-2994, Epub 23 March 2021<br>in particular, abstract | 1-44 |
| Y | SAIBIL, S. D. et al. Targeting T cell activation in immuno-oncology. Curr. Oncol. 2020, 27 (supply 2), S98-S105<br>in particular, p. S102, left column, 4th paragraph to right column, 3rd paragraph | 1-44 |
| Y | JP 2019-536469 A (THE UNITED STATES OF AMERICA, AS REPRESENTED BY THE SECRETARY, DEPARTMENT OF HEALTH AND HUMAN SERVICES) 19 December 2019 (2019-12-19)<br>claims, paragraphs [0003]-[0005], examples | 1-44 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 June 2022** | **21 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| | International application No. |
|---|---|
| | **PCT/JP2022/016625** |

**C.    DOCUMENTS CONSIDERED TO BE RELEVANT**

| Category* | Citation of document, with indication, where appropriate, of the relevant passages | Relevant to claim No. |
|---|---|---|
| A | US 2019/0092875 A1 (MEMORIAL SLOAN KETTERING CANCER CENTER) 28 March 2019 (2019-03-28) abstract, claims, examples | 1-44 |

<table>
<tr><td rowspan="2"><b>INTERNATIONAL SEARCH REPORT</b></td><td>International application No.</td></tr>
<tr><td><b>PCT/JP2022/016625</b></td></tr>
</table>

| **Box No. I** | **Nucleotide and/or amino acid sequence(s) (Continuation of item 1.c of the first sheet)** |

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

| International application No. |
|---|
| **PCT/JP2022/016625** |

| Patent document cited in search report | | | Publication date (day/month/year) | Patent family member(s) | | | Publication date (day/month/year) |
|---|---|---|---|---|---|---|---|
| WO | 2020/138489 | A1 | 02 July 2020 | US | 2022/0064312 | A1 | |
| | | | | claims, paragraphs [0001]-[003 8], [0120]-[0121], examples, fig. 2-7 | | | |
| | | | | JP | 2021-101710 | A | |
| | | | | EP | 3903817 | A1 | |
| | | | | KR | 10-2021-0108996 | A | |
| | | | | TW | 202039575 | A | |
| | | | | CN | 113260381 | A | |
| | | | | CA | 3124332 | A1 | |
| | | | | AU | 2019415395 | A1 | |
| JP | 2019-536469 | A | 19 December 2019 | US | 2019/0233526 | A1 | |
| | | | | claims, paragraphs [0003]-[000 5], examples | | | |
| | | | | JP | 2021-52804 | A | |
| | | | | US | 2021/0315985 | A1 | |
| | | | | WO | 2018/057585 | A1 | |
| | | | | EP | 3515475 | A1 | |
| | | | | CA | 3037518 | A1 | |
| | | | | AU | 2017332161 | A1 | |
| US | 2019/0092875 | A1 | 28 March 2019 | US | 2021/0364520 | A1 | |
| | | | | US | 10087259 | B1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10087259 B
- WO 2018181425 A
- WO 2018112032 A
- WO 2020138489 A
- WO 2021142002 A
- WO 2021152186 A
- WO 2021163064 A
- WO 2021178749 A
- WO 2021194942 A
- WO 2021260208 A
- WO 2021260209 A
- WO 2021260210 A

**Non-patent literature cited in the description**

- *Nat. Rev. Immunol,* 2006, vol. 6 (4), 295-307
- *Eur. J. Immunol.,* 2010, vol. 40, 3325-3335
- *J. Clin. Oncol,* 2006, vol. 24, 5373-5380
- *Nat. Med,* 2004, vol. 10, 942-949
- *J. Clin. Oncol,* 2007, vol. 25, 2586-2593
- *Cancer,* 2006, vol. 107, 2866-2872
- *Eur. J. Cancer,* 2008, vol. 44, 1875-1882
- *Cell. Mol. Immunol,* 2011, vol. 8, 59-66
- *J. Immunol.,* 1996, vol. 157 (7), 2759-63
- *J. Biol. Chem.,* 1997, vol. 272 (28), 17251-4
- *Cancer Res.,* 2016, vol. 76 (4), 4-04, 11
- *Immunity,* 2016, vol. 45, 1122-1134
- *Immunity,* 2016, vol. 45, 1135-1147
- *Proc. Natl. Acad. Sci. USA,* 2018, vol. 115 (45), 10672-10681
- **DANIEL O VILLARREAL et al.** Targeting of CCR8 induces antitumor activity as a monotherapy that is further enhanced in combination with a Listeria-based immunotherapy. *ASCO 2018, https://www.advaxis.com/wp-content/uploads/2018/04/KeystonePoster CCR8-combo-posterFINAL.pdf*
- *Cancer Res.,* 2018, vol. 78 (18), 5340-5348
- *Proc. Natl. Acad. Sci. USA,* 1993, vol. 90 (2), 720-724
- *Cell Res,* 2017, vol. 27, 38-58
- *Cell Stem Cell,* 2018, vol. 23 (2), 181-192
- *J. Hematol. Oncol,* 2019, vol. 12 (1), 49
- Immunochemistry in Practice. Blackwell Scientific Publiations
- Molecular Cloning, A Laboratory Manual. Cold Spring Harbor Laboratory Press, 2012
- Current Protocols Essential Laboratory Techniques. *Current Protocols,* 2012
- **SATO, K et al.** *Cancer Res.,* 1993, vol. 53, 851
- *Cancer Science,* 2020, vol. 111, 1478-1490
- *Mol. Ther. Nucleic Acids,* 2018, vol. 12, 793-804